(19) Europäisches Patentamt
European Patent Office
Office européen des brevets

(11) **EP 4 303 577 B1**

(12) **EUROPÄISCHE PATENTSCHRIFT**

(45) Veröffentlichungstag und Bekanntmachung des Hinweises auf die Patenterteilung:
**01.10.2025 Patentblatt 2025/40**

(51) Internationale Patentklassifikation (IPC):
**G01N 27/404** (2006.01) **G01K 7/00** (2006.01)
**G01N 33/497** (2006.01)

(21) Anmeldenummer: **23182568.8**

(52) Gemeinsame Patentklassifikation (CPC):
**G01N 27/404; G01K 13/00; G01N 33/497;**
A61B 5/082; G01N 27/4163

(22) Anmeldetag: **30.06.2023**

(54) **SENSOR-ANORDNUNG MIT EINEM ELEKTROCHEMISCHEN SENSOR UND EINEM TEMPERATUR-SENSOR UND VERFAHREN UNTER VERWENDUNG EINER SOLCHEN SENSOR-ANORDNUNG**

SENSOR ASSEMBLY COMPRISING AN ELECTROCHEMICAL SENSOR AND A TEMPERATURE SENSOR AND METHOD USING SUCH A SENSOR ASSEMBLY

ENSEMBLE CAPTEUR COMPRENANT UN CAPTEUR ÉLECTROCHIMIQUE ET UN CAPTEUR DE TEMPÉRATURE ET PROCÉDÉ UTILISANT UN TEL ENSEMBLE CAPTEUR

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **06.07.2022 DE 102022116825**

(43) Veröffentlichungstag der Anmeldung:
**10.01.2024 Patentblatt 2024/02**

(73) Patentinhaber: **Dräger Safety AG & Co. KGaA**
**23560 Lübeck (DE)**

(72) Erfinder:
• **Baesler, Malte**
**23558 Lübeck (DE)**
• **Reier, Tobias**
**23558 Lübeck (DE)**

(74) Vertreter: **Meyer-Gramann, Klaus Dieter**
**c/o Drägerwerk AG & Co. KGaA**
**Moislinger Allee 53/55**
**23558 Lübeck (DE)**

(56) Entgegenhaltungen:
DE-A1- 102020 115 804    GB-A- 1 432 898
JP-A- 2021 156 740       US-A- 3 518 179
US-A1- 2014 061 043

**Beschreibung**

Die Erfindung betrifft eine Sensor-Anordnung und ein Verfahren zum Analysieren eines Gases auf mindestens einen vorgegebenen Gas-Bestandteil, wobei die Sensor-Anordnung einen elektrochemischen Sensor mit einer Messelektrode und einer Gegenelektrode umfasst, wobei zwischen diesen beiden Elektroden ein Elektrolyt angeordnet ist und wobei das Verfahren unter Verwendung einer derartigen Sensor-Anordnung durchgeführt wird.

Derartige elektrochemische Sensoren werden beispielsweise in Analysegeräten für Alkohol verwendet. Ein solches Analysegerät untersucht den Gehalt von Atemalkohol, insbesondere von Ethanol, in einer Atemprobe, die ein Proband ausgeatmet hat. Der Anteil von Atemalkohol in der Atemprobe ist ein Maß für den Gehalt von Alkohol im Blut des Probanden.

In US 2015 / 0 076 007 A1 wird ein elektrochemischer Sensor mit einer Messelektrode (working electrode, sensing electrode), einer Gegenelektrode und einem Elektrolyten zwischen den beiden Elektroden beschrieben. An die Messelektrode lassen sich unterschiedlich hohe elektrische Spannungen anlegen, so dass unterschiedliche positive Potentiale der Messelektrode relativ zu einer Referenzelektrode auftreten. Zwei verschiedene elektrochemische Reaktionen werden herbeigeführt, nämlich eine Oxidierung oder eine Reduktion einer ersten chemischen Substanz sowie eine Oxidation oder Reduzierung einer zweiten chemischen Substanz. Ein Temperatur-Sensor misst ein erstes elektrisches Potential, bei dem die erste elektrochemische Reaktion stattfindet, und ein zweites elektrisches Potential, bei dem die zweite elektrochemische Reaktion stattfindet, und zwar beides bei gleicher Temperatur. Aus der Differenz zwischen den beiden gemessenen elektrischen Potentialen wird näherungsweise die Temperatur der Messelektrode hergeleitet.

US 3,518,179 A offenbart eine elektrochemische Sensorzelle mit einem an der Rückseite einer der Elektroden angebrachten Thermistor zur Temperaturkontrolle.

Der Erfindung liegt die Aufgabe zugrunde, eine Sensor-Anordnung zum Analysieren eines Gases bereitzustellen, wobei die Sensor-Anordnung eine Messelektrode, eine Gegenelektrode und einen Elektrolyten umfasst und eine höhere Zuverlässigkeit als bekannte derartige Sensor-Anordnungen aufweisen soll. Weiterhin liegt der Erfindung die Aufgabe zugrunde, ein Verfahren zum Analysieren eines Gases bereitzustellen, wobei das Verfahren unter Verwendung einer derartigen Sensor-Anordnung durchgeführt wird und eine höhere Zuverlässigkeit als bekannte Verfahren aufweisen soll.

Die Aufgabe wird durch eine Sensor-Anordnung mit den Merkmalen des Anspruchs 1 und durch ein Verfahren mit den Merkmalen des Anspruchs 9 gelöst. Vorteilhafte Ausgestaltungen der erfindungsgemäßen Sensor-Anordnung sind, soweit sinnvoll, auch vorteilhafte Ausgestaltungen des erfindungsgemäßen Verfahrens und umgekehrt.

Die erfindungsgemäße Sensor-Anordnung und das erfindungsgemäße Verfahren vermögen ein Gas auf mindestens einen vorgegebenen Gas-Bestandteil zu untersuchen. Das Gas ist beispielsweise Luft und insbesondere Atemluft, die ein Proband ausgeatmet hat, oder Umgebungsluft. Der vorgegebene Gas-Bestandteil ist bevorzugt oxidierbar und beispielsweise Ethanol, Kohlenmonoxid oder Methanol.

In einer Anwendung wird durch die Sensor-Anordnung und das Verfahren automatisch entschieden, ob die Menge und / oder die Konzentration (der Gehalt) des oder mindestens eines Gas-Bestandteils im Gas unterhalb oder oberhalb einer vorgegebenen Schranke liegt. In einer anderen Anwendung werden durch die Sensor-Anordnung und das Verfahren die Menge und / oder die Konzentration des oder mindestens eines Gas-Bestandteils im Gas wenigstens näherungsweise gemessen, optional die summierten Konzentrationen aller GasBestandteile.

Möglich ist auch, dass die Sensor-Anordnung und das Verfahren eine Menge des Gas-Bestandteils in einer Menge einer Probe des Gases messen, wobei die Menge oder das Volumen der Gasprobe bekannt sind, wobei aus der gemessenen Menge des Bestandteils und der bekannten Menge der Gasprobe die gesuchte Konzentration des Gas-Bestandteils hergeleitet werden.

Die erfindungsgemäße Sensor-Anordnung umfasst einen elektrochemischen Sensor. Dieser elektrochemische Sensor umfasst

- eine Messelektrode,
- eine Gegenelektrode,
- eine elektrische Kontaktierung für die Messelektrode,
- eine elektrische Kontaktierung für die Gegenelektrode und
- einen Elektrolyten zwischen der Messelektrode und der Gegenelektrode.

Die Sensor-Anordnung vermag eine Detektions-Größe des elektrochemische Sensors zu messen. Die gemessene Detektions-Größe ist bevorzugt eine elektrische Größe, beispielsweise eine elektrische Spannung zwischen den beiden elektrischen Kontaktierungen oder die Stärke eines elektrischen Stroms, der durch eine Verbindung zwischen den beiden elektrischen Kontaktierungen fließt, eine elektrische Ladung (Menge des fließenden Stroms) oder ein elektrischer Widerstand.

Die Detektions-Größe korreliert mit dem Vorhandensein und / oder der Menge und / oder der Konzentration des oder mindestens eines Gas-Bestandteils im Gas. Beispielsweise bewirkt der Gas-Bestandteil eine chemische Reaktion im oder am elektrochemischen Sensor, und die chemische Reaktion beeinflusst die Detektions-Größe, insbesondere die elektrische Spannung oder die Stärke eines elektrischen Stroms. Die gemessene Detektions-Größe ist daher ein Maß für die Menge des Gas-Bestandteils in einer bestimmten Menge des Gases und / oder für die Konzentration des Gas-Bestandteils im Gas. Die Detektions-Größe ist in der Regel umso größer oder auch umso kleiner, je größer die gesuchte Menge oder Konzentration des Gas-Bestandteils im Gas ist.

Die Sensor-Anordnung umfasst weiterhin eine Temperatur-Sensoreinheit, welche wenigstens näherungsweise die aktuelle Temperatur der Messelektrode und / oder die aktuelle Temperatur der Gegenelektrode zu ermitteln vermag. Die Temperatur-Sensoreinheit umfasst ein elektrisch leitendes Messelement und einen Temperatur-Sensor. Das Messelement umfasst ein Kontakt-Segment und ein Verbindungs-Segment. Diese beiden Segmente sind elektrisch und / oder thermisch leitend miteinander verbunden.

Das Kontakt-Segment steht in einem flächigen Kontakt mit einem Messobjekt des elektrochemischen Sensors. Dank dieses flächigen Kontakts ist ein thermischer Kontakt zwischen dem Kontakt-Segment und dem Messobjekt hergestellt. Idealerweise weisen das Kontakt-Segment und das Messobjekt dank des thermischen Kontakts die gleiche Temperatur auf. Unter dem Begriff "Messobjekt" der Sensor-Anordnung wird die Messelektrode, die Gegenelektrode, die elektrische Kontaktierung für die Messelektrode oder die elektrische Kontaktierung für die Gegenelektrode verstanden. Das Kontakt-Segment steht also in einem flächigen Kontakt mit der Messelektrode, mit der Gegenelektrode, mit der elektrischen Kontaktierung für die Messelektrode oder mit der elektrischen Kontaktierung für die Gegenelektrode. Möglich ist auch, dass dasselbe Kontakt-Segment in flächigem Kontakt mit zwei verschiedenen Messobjekten steht oder dass zwei verschiedene Kontakt-Segmente in flächigen Kontakt mit jeweils einem Messobjekt stehen, wobei dasselbe Verbindungs-Segment oder zwei verschiedene Verbindungs-Segmente mit den beiden Kontakt-Segmenten verbunden ist / sind.

Das Verbindungs-Segment verbindet das oder ein Kontakt-Segment elektrisch und / oder thermisch mit dem Temperatur-Sensor. Bevorzugt sind sowohl das Verbindungs-Segment als auch der Temperatur-Sensor räumlich sowohl von den beiden Elektroden als auch von den beiden elektrischen Kontaktierungen für diese Elektroden beabstandet.

Der Temperatur-Sensor vermag an einer Messposition eine Größe, die mit der Temperatur des Kontakt-Segments korreliert, zu messen. Diese gemessene Größe ist insbesondere eine Größe, die mit der Temperatur des Verbindungs-Segments an der Messposition korreliert. Die gemessene Größe kann auch direkt die Temperatur des Verbindungs-Segments an der Messposition sein. Die Messposition kann einen einzelnen Messpunkt oder zwei einzelne Messpunkte umfassen, beispielsweise zwei Messpunkte, zwischen denen eine elektrische Spannung auftritt, die gemessen wird.

Unter dem Begriff der "gemessenen Größe eines Segments" wird die korrelierende Größe dieses Segments an der Messposition verstanden. Die Messposition ist räumlich von beiden Elektroden und räumlich von beiden elektrischen Kontaktierungen beabstandet, bevorzugt auch räumlich vom Kontakt-Segment beabstandet.

Die Temperatur-Sensoreinheit vermag die aktuelle Temperatur der Messelektrode und / oder die aktuelle Temperatur der Gegenelektrode abhängig von der Größe, die mit der Temperatur des Kontakt-Segments korreliert und die an der Messposition gemessen wurde, zu ermitteln.

Das erfindungsgemäße Verfahren wird unter Verwendung einer derartigen Sensor-Anordnung durchgeführt und umfasst die folgenden Schritte:

- Die Detektions-Größe des elektrochemischen Sensors wird gemessen. Diese Detektions-Größe korreliert mit dem gesuchten Vorhandensein und / oder der Konzentration des oder mindestens eines Gas-Bestandteils im Gas.
- Der Temperatur-Sensor misst an der Messposition die Größe, die mit der Temperatur des Kontakt-Segments korreliert.
- Abhängig von der Größe, die mit der Temperatur des Kontakt-Segments korreliert, wird wenigstens näherungsweise die Temperatur der Messelektrode und / oder die Temperatur der Gegenelektrode ermittelt.

Erfindungsgemäß wird eine Detektionsgröße gemessen, wobei diese Detektions-Größe von der Menge und / oder Konzentration des Bestandteils im Gas abhängt. Die Detektions-Größe hängt aber häufig nicht nur von der Menge und / oder Konzentration des Bestandteils ab, sondern zusätzlich von der Temperatur der Messelektrode und / oder der Temperatur der Gegenelektrode. Diese Elektroden-Temperatur wird in der Regel von Umgebungsbedingungen beeinflusst, insbesondere von der Umgebungstemperatur und / oder der Temperatur in einer Messkammer der Sensor-Anordnung und in geringerem Maße auch von der Feuchtigkeit und der Temperatur des zu analysierenden Gases.

In vielen Fällen dauert die Analyse des Gases umso länger, je niedriger die Temperatur der Messelektrode ist. In manchen Fällen führt eine zeitlich lange Analyse zu einem relativ hohen Messrauschen. Insbesondere bei einer relativ niedrigen Umgebungstemperatur kann Feuchtigkeit in einer Fluidführungseinheit, beispielsweise in einem Schlauch, kondensieren, wobei diese Fluidführungseinheit zu dem elektrochemischen Sensor führt. Die kondensierte Feuchtigkeit kann die chemische Zusammensetzung des Gases verändern und daher ein Messergebnis des elektrochemischen Sensors verfälschen. Bei einer Messelektrode mit einer niedrigen Temperatur kann außerdem in manchen Fällen Feuchtigkeit an der Messelektrode kondensieren, was ebenfalls ein Messergebnis verfälschen kann. Außerdem können zu einem Zeitpunkt an unterschiedlichen Stellen im elektrochemischen Sensor verschiedene Temperaturen auftreten. Insbesondere um die gerade beschriebenen dynamischen Effekte zu erfassen und idealerweise rechnerisch zu kompensieren, wird gewünscht, die aktuelle Temperatur mindestens einer Elektrode des elektrochemischen Sensors ausreichend genau zu kennen.

Die Temperatur-Sensoreinheit vermag wenigstens näherungsweise die aktuelle Temperatur der Messelektrode und / oder die Temperatur der Gegenelektrode zu ermitteln. Die Kenntnis der Elektroden-Temperatur ermöglicht es in vielen Fällen, automatisch den Einfluss der Elektroden-Temperatur auf das Messergebnis bis zu einem gewissen Grad rechnerisch zu kompensieren und / oder die Elektroden-Temperatur zu regeln.

Erfindungsgemäß vermag der Temperatur-Sensor an einer Messposition eine Größe, die mit der Temperatur des Kontakt-Segments korreliert, zu messen. Dank des flächigen Kontakts und dem resultierenden thermischen Kontakt hat das Kontakt-Segment annähernd die gleiche Temperatur wie das Messobjekt. "Annähernd gleiche Temperatur" bedeutet, dass mögliche Temperaturunterschiede so gering sind, dass sie für Anwendungen der Erfindung vernachlässigt werden können, und / oder kleiner als eine vorgegebene Toleranz sind.

Das Messobjekt kann die oder eine Elektrode sein, deren Temperatur gemessen werden soll. Das Messobjekt kann auch die elektrische Kontaktierung für diese Elektrode sein. In der Regel hat die elektrische Kontaktierung für eine Elektrode annähernd die gleiche Temperatur wie die kontaktierte Elektrode selber. Daher lässt sich in vielen Fällen die Temperatur der Elektrode auch dann ausreichend genau messen, wenn das Kontakt-Segment die elektrische Kontaktierung für die Elektrode kontaktiert, aber von der Elektrode selber räumlich beabstandet ist. In manchen Anwendungen ist es leichter, das Kontakt-Segment in einen flächigen Kontakt mit der elektrischen Kontaktierung zu bringen als mit der Elektrode selber.

In manchen anderen Fällen lässt sich eine vorgegebene Rechenvorschrift auf die gemessene Größe anwenden, um die Temperatur der Elektrode herzuleiten, wobei diese Rechenvorschrift durch die Konstruktion der Sensor-Anordnung bedingt ist, weswegen die Anwendung der Rechenvorschrift keinen zusätzlicher Sensor benötigt.

Erfindungsgemäß vermag der Temperatur-Sensor an der Messposition eine Größe, die mit der Temperatur des Kontakt-Segments korreliert, zu messen. Die korrelierende Größe ist beispielsweise der elektrische Widerstand oder eine messbare Größe, die mit dem elektrischen Widerstand korreliert. Die Messposition kann in oder an dem Verbindungs-Segment liegen. Der Temperatur-Sensor vermag dann eine Größe zu messen, die mit der Temperatur des Verbindungs-Segments korreliert.

Erfindungsgemäß wird die Größe, die mit der Temperatur des Kontakt-Segments korreliert, an der räumlich entfernten Messposition gemessen. Dadurch ist es nicht erforderlich, die Temperatur an einer Messposition zu messen, die an oder auf oder in dem Messobjekt oder dem Kontakt-Segment liegt. Vielmehr trägt das Verbindungs-Segment dazu bei, den Abstand zwischen dem Messobjekt und dem Kontakt-Segment einerseits und der Messposition andererseits zu überbrücken.

Dank der Erfindung lässt sich die Temperatur mindestens einer Elektrode messen, während die Sensor-Anordnung und damit ein Analysegerät, welches die erfindungsgemäße Sensor-Anordnung umfasst, eingesetzt wird. Dank der Erfindung ist es nicht erforderlich, die Sensor-Anordnung in einen speziellen Modus zu versetzen, um die Elektroden-Temperatur zu messen. Weiterhin ist es dank der Erfindung nicht erforderlich, der Sensor-Anordnung eine chemische Substanz eigens zu dem Zweck zuzuführen, die Elektroden-Temperatur zu messen. In vielen Fällen erfordert die Messung der Elektroden-Temperatur nur relativ wenig Zeit, was häufig ermöglicht, die Elektroden-Temperatur rasch auf einen vorgegebenen Wert zu regeln.

Erfindungsgemäß vermag der Temperatur-Sensor die Größe, die mit der Kontakt-Segment-Temperatur korreliert, an der Messposition zu messen. Diese Messposition ist räumlich von beiden Elektroden und von beiden elektrischen Kontaktierungen beabstandet. Bevorzugt ist die gesamte Sensor-Anordnung oder wenigstens der elektrochemische Sensor im Inneren eines Gehäuses angeordnet. Die Messposition befindet sich bevorzugt im Inneren dieses Gehäuses. Dadurch wird die Gefahr verringert, dass das Gehäuse eine Messung verfälscht. Außerdem wird die Gefahr verringert, dass das Gehäuse zwischen dem elektrochemischen Sensor und der Messposition ein größerer Zeitverzug zwischen einer Temperaturänderung und der Detektion dieser Temperaturänderung auftritt.

Erfindungsgemäß vermag die Temperatur-Sensoreinheit die Temperatur der Messelektrode und / oder die Temperatur der Gegenelektrode zu messen, optional die jeweilige Temperatur beider Elektroden. Häufig umfasst der elektrochemische Sensor zusätzlich eine Referenzelektrode sowie eine elektrische Kontaktierung für die Referenzelektrode. In der Regel wird das elektrische Potenzial der Referenzelektrode konstant gehalten. Häufig erreicht idealerweise eine zu untersuchende Gasprobe nicht die Referenzelektrode. In der Regel hängt die Detektionsgröße zusätzlich von der Temperatur der Referenzelektrode ab.

In einer Ausgestaltung vermag die Temperatur-Sensoreinheit zusätzlich die Temperatur der Referenzelektrode zu messen. Das Kontakt-Segment des Messelements oder ein Kontakt-Segment eines weiteren Messelements steht dergestalt in einem flächigen Kontakt mit der Referenzelektrode oder der elektrischen Kontaktierung für die Referenzelektrode, dass ein thermischer Kontakt hergestellt ist. Der erfindungsgemäße oder ein weiterer Temperatur-Sensor vermag an einer räumlich beanstandeten Messposition eine Größe zu messen, die mit der Temperatur desjenigen Kontakt-Segments korreliert, welches die Referenzelektrode oder die elektrische Kontaktierung für die Referenzelektrode kontaktiert. In vielen Fällen ermöglicht diese Ausgestaltung es, auch die Temperatur der Referenzelektrode zu regeln und insbesondere konstant zu halten

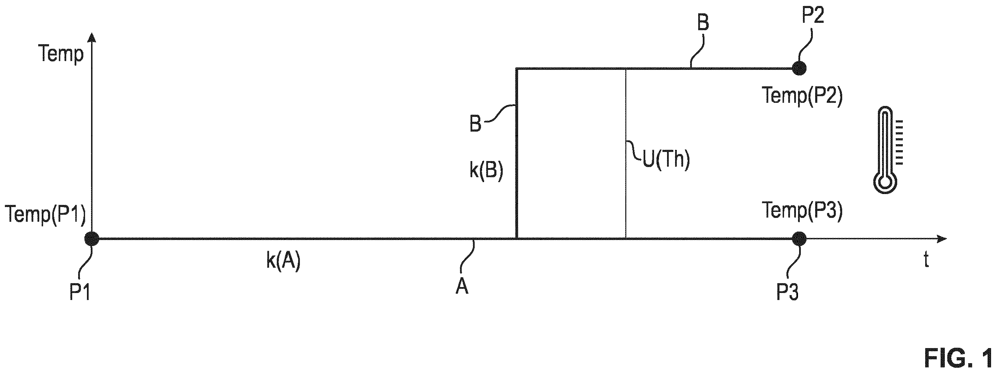

Erfindungsgemäß wird der sogenannte Seebeck-Effekt (thermoelektrische Effekt) ausgenutzt, um die Temperatur der Elektrode zu messen. Dieser Seebeck-Effekt wird zunächst allgemein mit Bezug auf Figur 1 erläutert.

Die Temperatur eines Objekts in einem Punkt P1 soll gemessen werden. Ein elektrischer Leiter A ist im Punkt P1 mit einem elektrischen Leiter B elektrisch verbunden. Die Temperaturen der beiden Leiter A und B in P1 und die gesuchte Objekt-Temperatur in P1 stimmen ausreichend genau überein. Die beiden Leiter A und B weisen zwei unterschiedliche Seebeck-Koeffizienten k(A) und k(B) auf. Die Eigenschaft "Seebeck-Koeffizient" wird auch als "thermische Leistung" oder "thermoelektrische Sensitivität" bezeichnet und ist eine werkstoffspezifische Konstante. Die Einheit ist bevorzugt Mikro-Volt pro Kelvin.

Gemäß dem Seebeck-Effekt tritt eine sogenannte Thermo-Spannung U(Th) auf. Diese Thermo-Spannung U(Th) tritt zwischen einem Punkt P3 auf dem Leiter A und einem Punkt P2 auf dem Leiter B auf, wobei zwischen P1 und P2, zwischen P1 und P3 sowie zwischen P2 und P3 jeweils ein Abstand auftritt. Beispielsweise erstreckt sich der Leiter A von P1 nach P3 und der Leiter B von P1 nach P2. Die Temperatur des Leiters A in P3 und die Temperatur des Leiters B in P2 werden als ausreichend übereinstimmend angenommen. Die Thermo-Spannung U(Th) hängt von den beiden Seebeck-Koeffizienten k(A) und k(B) ab, in der Regel von der Differenz k(A) - k(B), und außerdem von der Temperatur Temp(P1) in P1 und von der Temperatur Temp(P3) des Leiters A in P3, die mit der Temperatur Temp(P2) des Leiters B in P2 übereinstimmt. Die beiden Seebeck-Koeffizienten k(A) und k(B) sind konstruktionsbedingt bekannt, und die Thermo-Spannung U(Th) sowie die Temperatur Temp (P3) des Leiters A in P3 und / oder die Temperatur Temp(P2) des Leiters B in P2 werden gemessen. Dann lässt sich die gesuchte Temp(P1) herleiten. In vielen Fällen gilt mit ausreichender Genauigkeit:

$$U(Th) = [k(A) - k(B)] * [Temp(P1) - Temp(P2)] =$$

$$[k(A) - k(B)] * [Temp(P1) - Temp(P3)].$$

In dieser Rechenvorschrift ist Temp(P1) die einzige Unbekannte.

Gemäß der Erfindung wird der Seebeck-Effekt wie folgt ausgenutzt: Der Bereich, in dem das Kontakt-Segment in flächigem Kontakt mit dem Messobjekt steht, fungiert als der oder umfasst den Punkt P1. Mindestens im Punkt P1 ist das Kontakt-Segment zusätzlich elektrisch mit dem Messobjekt verbunden. Zu dem elektrischen Leiter A gehört ein Segment der elektrischen Kontaktierung für eine Elektrode. Als der elektrische Leiter B fungiert das elektrisch leitende Verbindungs-Segment. Die beiden Punkte P2 und P3 liegen an jeweils einer räumlich entfernten Referenz-Messposition, beispielsweise auf einer Platine, mit der die beiden Leiter A und B verbunden sind. Bevorzugt fungieren die beiden Punkte P2 und P3 zusammen als die räumlich beabstandete Messposition. Das elektrisch leitende Messelement oder wenigstens das Verbindungs-Segment weist einen anderen Seebeck-Koeffizienten auf als der elektrische Leiter A. In der Regel ist die Annahme gerechtfertigt, dass das Verbindungs-Segment durchgehend den gleichen Seebeck-Koeffizienten aufweist, optional sogar das gesamte Messelement, und der elektrische Leiter A ebenfalls durchgehend den gleichen Seebeck-Koeffizienten aufweist. Die beiden Seebeck-Koeffizienten sind aus der Konstruktion der Sensor-Anordnung bekannt oder lassen sich vorab empirisch ermitteln.

Die Temperatur-Sensoreinheit umfasst zusätzlich zum Temperatur-Sensor einen Spannungs-Sensor. Der Spannungs-Sensor ist räumlich von beiden Elektroden beabstandet und vermag ein Maß für die Thermo-Spannung U(Th) zwischen den Punkten P2 und P3 zu messen. Die gemessene Thermo-Spannung U(Th) tritt also zwischen den Verbindungs-Segment einerseits und dem Messobjekt oder der elektrischen Kontaktierung für das Messobjekt andererseits auf. Gemäß der Erfindung vermag der Temperatur-Sensor eine Größe, die mit der Temperatur des Verbindungs-Segments korreliert, zu messen. Die Temperatur-Sensoreinheit verwendet die Temperatur des Verbindungs-Segments als die Temperatur am Punkt P2 und / oder am Punkt P3 oder leitet die Temperatur am Punkt P2 oder am Punkt P3 aus der Temperatur des Verbindungs-Segments her. In vielen Fällen differieren die Temperaturen an den Punkten P2 und P3 nicht wesentlich voneinander. Die Temperatur-Sensoreinheit leitet die Temperatur am Punkt P1 aus

- der gemessenen Thermo-Spannung,
- der gemessene Temperatur am Punkt P2 und / oder P3 und
- den beiden Seebeck-Koeffizienten, bevorzugt der Differenz zwischen den beiden Seebeck-Koeffizienten,
 her.

Dies erspart die Notwendigkeit, direkt die Temperatur des Kontakt-Segments oder eine Größe, die mit der Temperatur des Kontakt-Segments korreliert, zu messen. Weiterhin ist es zwar möglich, aber nicht erforderlich, dass das Kontakt-Segment in einem guten thermischen Kontakt mit dem Verbindungs-Segment steht. Vielmehr wird gemäß der Erfindung eine Temperatur an einer räumlich entfernten Messposition gemessen, nämlich am Punkt P2 oder P3. In vielen Fällen steht am Punkt P2 oder am Punkt P3 wesentlich mehr Platz für einen Temperatur-Sensor zur Verfügung als am Punkt P1. Außerdem lässt sich die Temperatur oder eine mit der Temperatur korrelierende Größe am Punkt P2 oder am Punkt P3 mit größerer Zuverlässigkeit und / oder schneller messen als am Punkt P1. In vielen Fällen erspart dies außerdem die Notwendigkeit, das elektrisch leitende Messelement oder wenigstens das Kontakt-Segment vom Messobjekt elektrisch zu isolieren. Eine solche elektrische Isolierung erfordert Platz und kann undicht werden, also in unerwünschter Weise einen elektrischen Kontakt ermöglichen. Ein unerwünschter elektrischer Kontakt kann ein Messergebnis der Sensor-Anordnung verfälschen.

Nachfolgend wird eine weitere Ausführungsform der Erfindung beschrieben. Gemäß dieser Ausführung ist das Messobjekt die Messelektrode oder die Gegenelektrode. Das Kontakt-Segment kontaktiert also eine Elektrode. Das Verbindungs-Segment weist einen anderen Seebeck-Koeffizienten auf als die elektrische Kontaktierung der Elektrode, die als das Messobjekt fungiert. Der Spannungs-Sensor vermag ein Maß für folgende Thermo-Spannung zu messen: für die Thermo-Spannung, die zwischen dem Verbindungs-Segment und der elektrischen Kontaktierung des Messobjekts auftritt.

Gemäß dieser Ausführung kontaktiert das Kontakt-Segment des Messelements die Elektrode thermisch und elektrisch im Punkt P1. In vielen Fällen lässt sich die gesuchte Temperatur dieser Elektrode besser herleiten, als wenn das Kontakt-Segment die elektrische Kontaktierung der Elektrode und nicht die Elektrode selber kontaktieren würde. Die ohnehin vorhandene elektrische Kontaktierung der Elektrode wird zusätzlich dafür verwendet, um die Thermo-Spannung zu messen.

Gemäß der Erfindung, vermag der Temperatur-Sensor eine Größe, die mit der Temperatur des Verbindungs-Segments korreliert, zu messen. In einer Ausführungsform wird als das Messobjekt die Messelektrode oder die Gegenelektrode verwendet. Gemäß der Erfindung wird als die Größe, die mit der Temperatur des Verbindungs-Segments korreliert, die Temperatur der elektrischen Kontaktierung derjenigen Elektrode, die als das Messobjekt fungiert, gemessen. Wiederum wird die Thermo-Spannung zwischen dem Messelement und der elektrischen Kontaktierung gemessen. In manchen Fällen vergrößert diese Fortbildung die Zuverlässigkeit, mit der unter Verwendung des Seebeck-Effekts die gesuchte Temperatur der Elektrode ermittelt wird.

Erfindungsgemäß steht das Kontakt-Segment in einem thermischen Kontakt mit dem Messobjekt. Der Temperatur-Sensor vermag bevorzugt eine Größe, die mit der Temperatur des Kontakt-Segments korreliert, zu messen. In einer Ausgestaltung ist diese Größe eine Größe, die mit der Temperatur des Verbindungs-Segments korreliert. In manchen Fällen weicht die Temperatur des Verbindungs-Segments nicht in relevanter Weise von der Temperatur des Messobjekts ab. In anderen Fällen lässt sich die Temperatur des Kontakt-Segments und dadurch die Temperatur der Elektrode aus der Temperatur des Verbindungs-Segments herleiten, beispielsweise so wie gerade beschrieben durch Ausnutzung des Seebeck-Effekts.

Um zu verhindern, dass das Kontakt-Segment ein Messergebnis des elektrochemischen Sensors verfälscht, ist gemäß einer nichterfindungsgemäßen

Ausgestaltung der Erfindung das Kontakt-Segment elektrisch vom Messobjekt isoliert, beispielsweise durch eine isolierende Ummantelung um das Kontakt-Segment. Das Verbindungs-Segment ist räumlich von beiden Elektroden und von beiden elektrischen Kontaktierungen beabstandet und braucht daher nicht notwendigerweise elektrisch isoliert zu werden. Dank des thermischen Kontakts zwischen dem Messobjekt und dem Kontakt-Segment weist das Kontakt-Segment mit ausreichender Genauigkeit die gleiche Temperatur auf wie das Messobjekt, und zwar trotz der elektrischen Isolierung. Besonders bevorzugt ist das Messobjekt die Messelektrode oder die Gegenelektrode. Der Temperatur-Sensor vermag an der räumlich beabstandeten Messposition die Größe, die mit der Temperatur des Kontakt-Segments korreliert, zu messen. Die Messposition befindet sich beispielsweise an dem Verbindungs-Segment.

Die gerade beschriebene alternative nicht-erfindungsgemäße Ausgestaltung erspart die Notwendigkeit, zwei elektrisch leitende Bauteile mit unterschiedlichen Seebeck-Koeffizienten vorzusehen. Außerdem ist nicht notwendigerweise ein Spannungs-Sensor erforderlich. Der Temperatur-Sensor ist räumlich vom Messobjekt und vom Kontakt-Segment beabstandet.

In einer Ausgestaltung wird als die Größe, die mit der Temperatur des Kontakt-Segments korreliert, ein Maß für den elektrischen Widerstand des Kontakt-Segments gemessen. Beispielsweise wird wenigstens zeitweise ein elektrischer Stromkreis erzeugt, der das Kontakt-Segment umfasst. Die elektrische Spannung, die am Kontakt-Segment anlegt, sowie die Stärke des Stroms, der durch das Kontakt-Segment fließt, werden gemessen. Bekanntlich korreliert der elektrische Widerstand eines elektrisch leitenden Elements mit dessen Temperatur. Weil der elektrische Widerstand des Kontakt-Segments gemessen wird, ist es in vielen Fällen nicht erforderlich, das Messobjekt elektrisch zu kontaktieren, um den elektrischen Widerstand zu messen. Dies reduziert die Gefahr, dass die Messung des elektrischen Widerstands ein Messergebnis des elektrochemischen Sensors verfälscht.

In einer bevorzugten Ausgestaltung umfasst die erfindungsgemäße Sensor-Anordnung zusätzlich eine ansteuerbare Heizung. Die angesteuerte und dadurch aktivierte Heizung vermag die Messelektrode und / oder die Gegenelektrode zu erwärmen.

Gemäß der Ausgestaltung mit der Heizung vermag ein signalverarbeitendes Steuergerät der Sensor-Anordnung die tatsächliche Temperatur der Messelektrode und / oder die tatsächliche Temperatur der Gegenelektrode zu regeln. Das Regelungsziel bei dieser Regelung ist, dass die tatsächliche Elektroden-Temperatur in einem vorgegebenen Temperaturbereich bleibt. Um die tatsächliche Elektroden-Temperatur zu steigern und dadurch die Regelabweichung zu verringern, vermag das Steuergerät die Heizung anzusteuern und dadurch zu aktivieren. Um das Regelungsziel zu erreichen und hierfür die Heizung anzusteuern, verwendet das Steuergerät ein Signal der Temperatur-Sensoreinheit. Das Steuergerät vermag die Heizung auch wieder zu deaktivieren.

Eine vorteilhafte Ausgestaltung des erfindungsgemäßen Verfahrens wird unter Verwendung einer ansteuerbaren Heizung und eines signalverarbeitenden Steuergeräts durchgeführt und umfasst den zusätzlichen Schritt, dass die tatsächliche Temperatur der Messelektrode und / oder die der Gegenelektrode automatisch geregelt wird. Das Regelungsziel bei dieser Regelung (closed-loop control) ist, dass die tatsächliche Temperatur der Elektrode in einem vorgegebenen Temperaturbereich bleibt. Für die Regelung verwendet das Steuergerät ein Signal der Temperatur-Sensoreinheit.

Dann, wenn die gemessene tatsächliche Temperatur unterhalb des Temperaturbereichs liegt, aktiviert das Steuergerät die Heizung. Die aktivierte Heizung erwärmt die Elektrode. Später, nämlich wenn die tatsächliche Temperatur wieder im Temperaturbereich liegt, deaktiviert das Steuergerät wieder die Heizung.

Die bevorzugte Ausgestaltung, dass die Elektroden-Temperatur automatisch geregelt wird, hat insbesondere die nachfolgend beschriebenen Vorteile.

Bei einer sehr hohen Temperatur der Messelektrode ist die Gefahr größer, dass die Messelektrode oder ein sonstiger Bestandteil der Sensor-Anordnung thermisch beschädigt wird, dass ein Teil des Elektrolyten verdampft und / oder sich auf der Messelektrode Ablagerungen bilden. Außerdem wird in manchen Fällen viel elektrische Energie verbraucht, was insbesondere dann von Nachteil ist, wenn die Sensor-Anordnung nicht dauerhaft mit einem stationären Spannungsversorgungsnetz verbunden werden kann, sondern ein Analysegerät mit der erfindungsgemäßen Sensor-Anordnung eine eigene Spannungsversorgungseinheit umfasst. Das Analysegerät kann ein tragbares Gerät sein.

Dank der erfindungsgemäßen Regelung wird die Temperatur der Messelektrode in dem vorgegebenen Temperaturbereich gehalten. Sowohl die Nachteile einer sehr niedrigen als auch die Nachteile einer sehr hohen Temperatur werden vermieden. Verglichen mit einer Ausgestaltung ohne eine Temperaturregelung wird dadurch die Zeit verringert, die zum Analysieren des Gases erforderlich ist. Außerdem wird bis zu einem gewissen Grad der Einfluss von Umgebungsbedingungen auf das Analyseergebnis rechnerisch kompensiert. Nicht erforderlich ist, ein Maß für eine Umgebungsbedingung zu messen, beispielsweise für die Umgebungstemperatur.

In einer Ausgestaltung wird direkt die Temperatur der Messelektrode geregelt, in einer anderen Ausgestaltung die Temperatur der Gegenelektrode, welche die Temperatur der Messelektrode beeinflusst. Verhindert werden sowohl eine zu niedrige als auch eine zu hohe Temperatur der Messelektrode. Möglich ist auch, dass sowohl die Temperatur der Messelektrode als auch die Temperatur der Gegenelektrode geregelt werden. Bevorzugt wird daher die Heizung nur so lange wie nötig eingeschaltet. Möglich ist auch, zusätzlich die Temperatur der optionalen Referenzelektrode zu regeln.

Erfindungsgemäß steht das Kontakt-Segment in einem thermischen Kontakt mit dem Messobjekt. In einer Ausgestaltung ist das Messobjekt die Messelektrode oder die Gegenelektrode. Auch dann, wenn das Messobjekt eine elektrische Kontaktierung ist, weist das Messobjekt in der Regel annähernd die gleiche Temperatur auf wie die Messelektrode und die Gegenelektrode. Einerseits ist die Messposition räumlich von beiden Elektroden und von beiden elektrischen Kontaktierungen beabstandet. Andererseits befindet sich die Messposition ausreichend nahe an mindestens einer Elektrode und insbesondere innerhalb eines Gehäuses. Bei einer weiter von der Elektrode entfernten Messposition könnte hingegen ein größerer Zeitverzug zwischen einer Temperaturänderung und einer entsprechenden Reaktion des Steuergeräts stattfinden. Diese Gefahr ist insbesondere dann gegeben, wenn die Sensor-Anordnung sich innerhalb eines Gehäuses befindet und die Messposition außen am Gehäuse oder sogar mit einem Abstand zum Gehäuse angeordnet ist. Außerdem ist bei einem großen Abstand zwischen der Messposition und der Elektrode die Gefahr größer, dass eine Störgröße in Form einer weiteren Wärmequelle oder Kältequelle die Temperaturmessung verfälscht.

Die Temperatur-Sensoreinheit liefert dank insbesondere dank des Merkmals mit dem Kontakt-Segment ein Signal für die aktuelle Temperatur des Messobjekts, wobei dieses Signal Temperaturänderungen des Messobjekts rasch folgt. Das Steuergerät vermag daher rasch auf eine Temperaturänderung des Messobjekts und damit einer Elektrode zu reagieren. Dank dieser raschen Reaktion wird in vielen Fällen erreicht, dass die tatsächliche Elektroden-Temperatur nur für sehr kurze Zeitspannen außerhalb des vorgegebenen Temperaturbereichs liegt.

In einer Ausgestaltung vermag der Temperatur-Sensor sowohl die tatsächliche Temperatur der Messelektrode als auch die tatsächliche Temperatur der Gegenelektrode zu messen. In einer Fortbildung der Ausgestaltung mit der Heizung vermag ein Bestandteil der Heizung die Messelektrode zu erwärmen, ein anderer Bestandteil die Gegenelektrode. Diese beiden Bestandteile lassen sich bevorzugt unabhängig voneinander ansteuern. Abhängig von der gemessenen tatsächlichen Temperatur der Messelektrode steuert das Steuergerät den Bestandteil zum Erwärmen der Messelektrode an, abhängig von der tatsächlichen Temperatur der Gegenelektrode den Bestandteil zum Erwärmen der Gegenelektrode. Möglich ist, dass für die Messelektrode und für die Gegenelektrode der gleiche gewünschte Temperaturbereich vorgegeben wird. Möglich ist auch, dass unterschiedliche Temperaturbereiche vorgegeben werden.

In einer Realisierungsform der Ausgestaltung mit der Heizung umfasst die Heizung eine ansteuerbare Strahlungsquelle. Die Strahlungsquelle vermag eine elektromagnetische Strahlung, insbesondere Infrarotstrahlung, in Richtung der Messelektrode und / oder der Gegenelektrode zu emittieren. Das Steuergerät vermag zu bewirken, dass die Intensität und / oder die Energie der Strahlung, welche von der Strahlungsquelle emittiert wird, auf einen vom Steuergerät berechneten Wert eingestellt wird. Um diesen Wert zu berechnen, verwendet das Steuergerät ein Signal der Temperatur-Sensoreinheit.

Erfindungsgemäß steht das Kontakt-Segment der Messeinheit in einem thermischen Kontakt mit dem Messobjekt. Gemäß der gerade beschriebenen Ausgestaltung vermag eine Heizung die Messelektrode und / oder die Gegenelektrode zu heizen. In einer Ausgestaltung ist das Messobjekt zugleich die beheizte Elektrode. Die Heizung umfasst ein elektrisch leitendes Heizelement. Dieses elektrische Heizelement umfasst das Kontakt-Segment und / oder stellt das Kontakt-Segment bereit. Dieses Kontakt-Segment steht in einem thermischen Kontakt mit den Messobjekt, hier also mit der beheizten Elektrode. Das Kontakt-Segment ist bevorzugt elektrisch von der beheizten Elektrode isoliert, um die Gefahr zu reduzieren, dass durch einen elektrischen Kontakt ein Messergebnis des elektrochemischen Sensors verfälscht wird.

Gemäß dieser Ausgestaltung hat das elektrische elektrisch leitende Heizelement also zwei Funktionen: Zum einen misst der Temperatur-Sensor eine Größe, die mit der aktuellen Temperatur des Heizelements korreliert, und die Temperatur-Sensoreinheit leitet aus der gemessenen Größe die Temperatur der kontaktierten Elektrode ab. Die Kenntnis der Heizelement-Temperatur wird bevorzugt dafür verwendet, um die Heizung zu steuern oder zu regeln. Zum anderen heizt dasselbe Heizelement die Elektrode. Die Ausgestaltung erspart also die Notwendigkeit, ein Kontakt-Segment und zusätzlich eine räumlich beabstandetes Heizelement vorzusehen.

Gemäß dieser Ausgestaltung wird eine Größe gemessen, die mit der Temperatur des Kontakt-Segments korreliert. In einer Realisierungsform misst die Sensoreinheit ein Maß für den elektrischen Widerstand des elektrisch leitenden Heizelements. Bekanntlich korreliert bei einem elektrisch leitenden Element der elektrische Widerstand mit der Temperatur des Elements.

Gemäß einer bevorzugten Realisierungsform der Ausgestaltung mit der Heizung berechnet das Steuergerät einen Sollwert für die elektrische Spannung, die an der Heizung anliegen soll. Dieser Wert für die elektrische anliegende elektrische Spannung legt die Temperatur fest, welche die Heizung an die Elektrode abgibt. Um den Sollwert für die elektrische Spannung zu berechnen, verwendet das Steuergerät ein Signal der Temperatur-Sensoreinheit. Das Steuergerät steuert die Heizung mit dem Ziel an, dass die tatsächlich an der Heizung anliegende elektrische Spannung gleich dem Sollwert ist.

Bevorzugt steuert das Steuergerät die Heizung an und aktiviert dann die Heizung, wenn die gemessene tatsächliche Temperatur der Messelektrode und / oder der Gegenelektrode unterhalb der unteren Grenze des vorgegebenen Temperaturbereichs liegt. In einer Ausgestaltung reicht eine einseitige Regelung der tatsächlichen Elektroden-Temperatur aus, weil die tatsächliche Elektroden-Temperatur nicht oder nicht in einem erheblichen Maß größer ist als die obere Schranke des Temperaturbereichs. Dies ist insbesondere dann der Fall, wenn die Temperatur der Elektrode kleiner oder nicht wesentlich größer als die Umgebungstemperatur ist. Außerdem ist in vielen Fällen das Ziel, eine zu niedrige Temperatur zu vermeiden, wichtiger als das Ziel, eine zu hohe Temperatur zu vermeiden, denn eine zu niedrige Temperatur beeinträchtigt die Empfindlichkeit und Zuverlässigkeit der Sensor-Anordnung häufig stärker als eine zu hohe Temperatur.

In einer Fortbildung dieser Ausgestaltung wird eine einseitige Regelung durchgeführt, also lediglich geheizt oder nicht geheizt. In einer anderen Fortbildung dieser Ausgestaltung vermag das Steuergerät zusätzlich eine Kühlung, d.h. ein Kühlelement, der Sensor-Anordnung anzusteuern. Wenn die gemessene tatsächliche Temperatur oberhalb des vorgegebenen Temperaturbereichs liegt, so aktiviert das Steuergerät die Kühlung. Später, nämlich dann, wenn die tatsächliche Temperatur wieder im Temperaturbereich liegt, so deaktiviert das Steuergerät die Kühlung.

In einer Ausgestaltung umfasst die Sensor-Anordnung also zusätzlich eine ansteuerbare Kühlung. Diese Kühlung vermag die Messelektrode und / oder die Gegenelektrode zu kühlen. Das Steuergerät steuert die Kühlung an und aktiviert dadurch die Kühlung dann, wenn die tatsächliche Temperatur oberhalb des vorgegebenen Temperaturbereichs liegt. Das Steuergerät vermag die Kühlung auch wieder zu deaktivieren.

In einer Ausgestaltung gehört die erfindungsgemäße Sensor-Anordnung zu einem Analysegerät, bevorzugt zu einem tragbaren Analysegerät. Das Gas, welches die Sensor-Anordnung zu analysieren vermag, ist bei einer Anwendung der Erfindung ein Teil einer Atemprobe, die ein Proband in ein Mundstück oder eine sonstige Eingabeeinheit des Analysegeräts eingibt. Der Gas-Bestandteil, auf den das Gas zu analysieren ist, ist Atemalkohol, insbesondere Ethanol, oder eine sonstige Substanz, die sich in der ausgeatmeten Atemluft eines Probanden befinden kann und die sich nachweisen lässt. In einer Fortbildung dieser Ausgestaltung misst die Sensor-Anordnung die Konzentration von Atemalkohol in der Atemprobe und leitet aus dieser Atemalkohol-Konzentration den Gehalt von Alkohol im Blut des Probanden ab. Bevorzugt gibt das Analysegerät den Alkoholgehalt in einer von einem Menschen wahrnehmbaren Form aus.

Im Folgenden wird die Erfindung anhand eines Ausführungsbeispiels beschrieben. Hierbei zeigt

Figur 1 schematisch den Seebeck-Effekt;
Figur 2 schematisch die Wirkungsweise eines elektrochemischen Sensors;
Figur 3 einen erfindungsgemäßen Regelkreis;
Figur 4 wie die Temperatur der Messelektrode mithilfe eines Thermoelements mit einem Kontakt-Segment an der Messelektrode gemessen wird;
Figur 5 eine Abwandlung der Ausgestaltung von Figur 4, wobei das Kontakt-Segment an der elektrischen Kontaktierung der Messelektrode angeordnet ist;
Figur 6 wie die Temperatur der Messelektrode mithilfe eines geraden Messdrahts an einer Stirnfläche gemessen wird (nicht-erfindungsgemäßes Beispiel);
Figur 7 wie die Temperatur der Messelektrode mithilfe eines gewickelten Messdrahts an der Stirnfläche gemessen wird (nicht-erfindungsgemäßes Beispiel);
Figur 8 wie die Messelektrode mithilfe einer Anordnung von LEDs geheizt wird.

Im Ausführungsbeispiel ist die erfindungsgemäße Sensor-Anordnung ein Bestandteil eines Analysegeräts, wobei der Rest des Analysegeräts in den Figuren nicht gezeigt wird. Das Analysegerät mit der Sensor-Anordnung wird dafür verwendet, um eine Atemprobe eines Probanden auf eine vorgegebene Substanz, insbesondere auf Atemalkohol, zu analysieren. Im Falle von Atemalkohol als der Substanz soll in einer Anwendung der Proband daraufhin untersucht werden, ob sich in seinem Blut Alkohol oberhalb einer vorgegebenen Nachweisgrenze befindet oder nicht. In einer anderen Anwendung soll der Gehalt von Alkohol in seinem Blut gemessen werden. In einer Ausgestaltung kann der Proband das Analysegerät in einer Hand halten. Das Analysegerät umfasst bevorzugt eine eigene Spannungsversorgungseinheit.

Der Proband gibt eine Atemprobe in ein Mundstück des Analysegeräts ein. Falls der Proband ein Getränk oder auch eine Speise zu sich genommen hat, die eine relevante Menge von Alkohol enthält, so enthält die abgegebene Atemprobe Atemalkohol, insbesondere gasförmiges Ethanol. Im Folgenden wird die Bezeichnung "Atemalkohol" für eine mögliche gasförmige Substanz verwendet, auf die das Analysegerät mit der erfindungsgemäßen Sensor-Anordnung eine Atemprobe untersuchen soll, also für den vorgegebenen Gas-Bestandteil.

Ein Teil der abgegebenen Atemprobe fließt in eine Messkammer des Analysegeräts. Dieser Teil wird im Folgenden als "Messkammer-Probe" bezeichnet. Ein elektrochemischer Sensor in oder an der Messkammer misst den Gehalt oder die Menge von Atemalkohol oder von einer sonstigen vorgegebenen Substanz in dieser Messkammer-Probe. Die nachfolgende Beschreibung bezieht sich auf Atemalkohol als der Substanz. Die Erfindung lässt sich insbesondere auch für eine andere Substanz verwenden, die in der ausgeatmeten Luft eines Probanden oder auch in der Umgebungsluft enthalten sein kann.

Der elektrochemische Sensor vermag ein Signal zu erzeugen, welches mit der Konzentration von Atemalkohol in der Messkammer-Probe, welche in der Messkammer ist, korreliert.

Aus dem Stand der Technik sind unterschiedliche geeignete elektrochemische Sensoren bekannt.

Das Analysegerät leitet aus der Menge oder Konzentration von Atemalkohol in der Messkammer-Probe sowie dem Volumen der Messkammer-Probe die Konzentration von Atemalkohol in der eingegebenen Atemprobe ab. Das Volumen der Messkammer-Probe wird beispielsweise abhängig von dem Volumen der Messkammer, welches durch die Konstruktion des Analysegeräts bekannt ist, und / oder einem gemessenen und aufintegrierten Volumenfluss in die Messkammer hergeleitet. Das Analysegerät oder eine räumlich entfernte Auswerteeinheit leitet aus der Atemalkohol-Konzentration oder der Atemalkohol-Menge in der Atemprobe den Gehalt von Alkohol im Blut des Probanden ab. Natürlich kann die Analyse zu dem Ergebnis führen, dass im Blut des Probanden kein Alkohol oberhalb einer Nachweisgrenze vorhanden ist.

Während die Atemprobe durch das Mundstück hindurch fließt, fließt zunächst Luft aus der Mund, dann Luft aus den oberen Atemwegen und anschließend Luft aus der Lunge des Probanden durch das Mundstück. Um festzustellen, ob das Blut des Probanden Alkohol enthält, ist ein Gas aus demjenigen Teil der Atemprobe zu untersuchen, der aus der Lunge stammt. Idealerweise fließt nur Gas, welches aus der Lunge des Probanden stammt, in die Messkammer, und die Messkammer-Probe enthält nur Luft aus der Lunge. Der restliche Teil der Atemprobe fließt aus Öffnungen des Mundstücks heraus, ohne die Messkammer zu erreichen. Ein Mundstück mit derartigen Öffnungen wird beispielsweise in DE 10 2017 008 008 A1 beschrieben.

In einer Ausgestaltung umfasst das Analysegerät eine Pumpe oder eine sonstige Fluidfördereinheit. Diese Fluidfördereinheit wird eingeschaltet, nachdem der Proband damit begonnen hat, die Atemprobe in das Mundstück einzugeben. Idealerweise saugt die Pumpe denjenigen Teil der Atemprobe ein, der aus der Lunge des Probanden stammt. Bevorzugt spült die Fluidfördereinheit die Messkammer wieder aus, nachdem der elektrochemische Sensor die Messkammer-Probe analysiert hat. Dadurch lässt sich dasselbe Analysegerät rasch hintereinander für mehrere Atemproben verwenden. Möglich ist auch, dass die Messkammer-Probe durch Diffusion in die Messkammer hinein fließt, ohne dass eine Fluidfördereinheit verwendet wird.

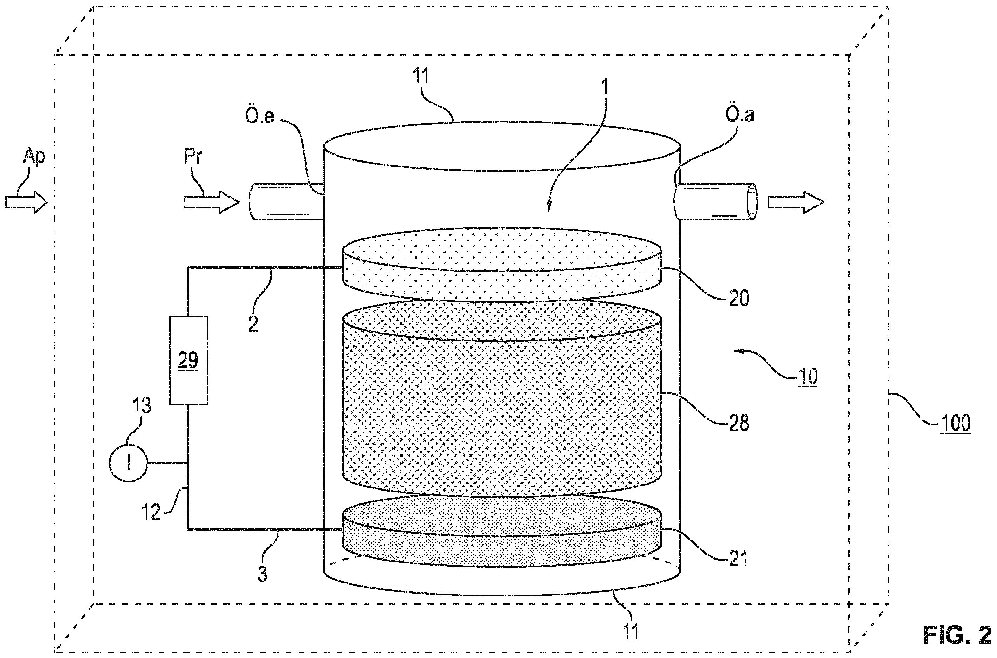

Das Analysegerät des Ausführungsbeispiels umfasst eine Sensor-Anordnung 100 mit einem elektrochemischen Sensor 10. Figur 2 zeigt schematisch die Wirkungsweise eines elektrochemischen Sensors 10, wie er aus dem Stand der Technik bekannt ist. Die Darstellung von Figur 2 ist nicht notwendigerweise maßstabsgerecht. Das erfindungsgemäße Analysegerät umfasst in einer Ausgestaltung eine derartige Sensor-Anordnung 100 und nimmt eine Atemprobe Ap auf.

Der elektrochemische Sensor 10 umfasst ein Gehäuse 11, das eine Messkammer 1 umschließt. Eine zu untersuchende Messkammer-Probe Pr fließt durch eine eintrittsseitige Öffnung Ö.e hindurch in das Innere des Gehäuses 11 und dort zur Messkammer 1, beispielsweise durch Diffusion oder indem die Messkammer-Probe Pr aktiv durch die Öffnung Ö.e hindurch in das Innere des Gehäuses 11 gesaugt wird. Die Messkammer-Probe Pr fließt durch eine austrittsseitige Öffnung Ö.a wieder aus der Messkammer 1 und dann aus dem Gehäuse 11 hinaus. Daher kann derselbe Sensor 10 nacheinander mehrere Messkammer-Proben Pr untersuchen.

Der elektrochemische Sensor 10 umfasst

- eine Messelektrode 20, die durch einen Kontaktierungsdraht 2 elektrisch kontaktiert ist,
- eine Gegenelektrode 21, die durch einen Kontaktierungsdraht 3 elektrisch kontaktiert ist,
- einen Elektrolyten 28 zwischen den beiden Elektroden 20 und 21,
- einen Verbindungsdraht 12, der die beiden Kontaktierungsdrähte 2 und 3 elektrisch miteinander verbindet und einen elektrischen Messwiderstand 29 umfasst, und
- einen Stromstärken-Sensor 13, der die Stärke des durch den Verbindungsdraht 12 fließenden Stroms misst.

Ein solcher elektrochemischer Sensor 10 wird nachfolgend auch als Membran-Elektroden-Elektrolyt-Einheit (MEEE) bezeichnet.

Der Elektrolyt 28 ist ein elektrisch leitendes Medium, beispielsweise mit Wasser verdünnte Schwefelsäure oder Phosphorsäure oder Perchlorsäure. In dem Elektrolyten 28 können sich Ionen bewegen. Bevorzugt stellt eine Membrane den Elektrolyten 28 bereit. Der Elektrolyt 28 stellt eine ionisch leitfähige Verbindung zwischen der Messelektrode 20 und der Gegenelektrode 21 her, verhindert aber einen Kurzschluss zwischen den beiden Elektroden 20 und 21.

Der Sensor 10 ist so ausgestaltet, dass die Messkammer-Probe Pr nur die Messelektrode 20 erreicht, aber nicht die Gegenelektrode 21. Im gezeigten Beispiel befindet sich die Messelektrode 20 an einer Wand der Messkammer 1, und das Gehäuse 11 und der Elektrolyt 28 verhindern, dass eine relevante Menge der Messkammer-Probe Pr die Gegenelektrode 21 erreicht.

Die beiden Kontaktierungsdrähte 2 und 3 sind elektrisch leitend und aus einem Material, welches vom Elektrolyten 28 nicht chemisch angegriffen wird, beispielsweise aus Platin oder Gold. Auch die Elektroden 20 und 21 sind aus einem chemisch resistenten Material, beispielsweise ebenfalls aus Platin oder Gold. In vielen Fällen fungiert das chemisch resistente Material zusätzlich als Katalysator für eine chemische Reaktion, die zur Messung hervorgerufen und verwendet wird.

In einer Realisierungsform funktioniert der elektrochemische Sensor 10 nach dem Prinzip der Brennstoffzelle. Die chemische Reaktion, die zur Messung verwendet wird, umfasst den Schritt, dass in der Messkammer 1 der Atemalkohol in der Messkammer-Probe Pr oxidiert wird. Idealerweise wird die gesamte Menge des Atemalkohols in der Messkammer-Probe Pr oxidiert. Als Folge der chemischen Reaktion tritt eine elektrische Spannung zwischen der Messelektrode 20 und der Gegenelektrode 21 auf, und daher fließt ein elektrischer Strom durch den Verbindungsdraht 12. Der Stromstärken-Sensor 13 misst die Stromstärke I und damit ein Maß für die elektrische Ladung, also die gesamte Menge des elektrischen Stroms, der durch den Verbindungsdraht 12 fließt (Prinzip der Coulometrie). Bekanntlich ist die elektrische Ladung das Integral der Stromstärke über die Zeit. Bei einem gegebenen Volumen der Messkammer-Probe Pr in der Messkammer 1 ist die gemessene elektrische Ladung umso höher, je mehr Atemalkohol die Messkammer-Probe Pr vor dem Oxidieren enthält. Die gemessene elektrische Ladung ist daher ein Maß für die Menge von Atemalkohol in der Messkammer-Probe Pr und damit für den Atemalkohol-Gehalt in der Atemprobe A und für den Gehalt an Alkohol im Blut des Probanden. Die elektrische Ladung ist also die Detektions-Größe des Ausführungsbeispiels.

Der elektrochemische Sensor 10 liefert ein elektrisches Signal, welches ein Maß für den Gehalt an Atemalkohol in der Messkammer-Probe Pr ist. Dieses Signal hängt aber nicht nur von der Menge oder der Konzentration von Atemalkohol in der Messkammer-Probe Pr ab, sondern wird auch von der Temperatur der beiden Elektroden 20 und 21 beeinflusst. Damit der Sensor 10 ein zuverlässiges Messergebnis liefern kann, wird im Ausführungsbeispiel die Temperatur der Elektroden 20, 21 mit dem Regelungsziel geregelt, dass die Temperatur in einem vorgegebenen Temperaturbereich bleiben soll. Ein Sonderfall ist, dass eine konstante Solltemperatur vorgegeben und die Temperatur der Elektroden 20, 21 mit dem Regelungsziel geregelt wird, dass die Temperatur konstant gleich dieser Solltemperatur sein soll. Eine Regelung (closed-loop control) und nicht nur eine Steuerung (open-loop control) der Elektroden-Temperatur wird durchgeführt, weil die Temperatur der Elektroden 20, 21 insbesondere von der Umgebungstemperatur beeinflusst wird. Möglich, aber dank der Regelung nicht erforderlich ist es, direkt die Umgebungstemperatur zu messen.

Bevorzugt umfasst der vorgegebene Temperaturbereich die typische Temperatur einer Atemprobe, die ein Mensch abgibt. Diese durchschnittliche Temperatur liegt zwischen 32 °C und 38 °C, besonders bevorzugt gleich 35 °C. Aus folgenden Gründen sollte der Temperaturbereich nicht zu niedrig liegen:

- Je höher die Temperatur der Elektroden 20, 21 ist, desto schneller liefert der Sensor 10 ein Ergebnis. Die elektrochemische Reaktion läuft nämlich umso schneller ab, je höher die Temperatur ist. Ein geringer Zeitbedarf, um das Ergebnis zu liefern, ist insbesondere dann wichtig, wenn dasselbe Analysegerät nacheinander zum Untersuchen mehrerer Atemproben auf Atemalkohol verwendet werden soll.
- Je mehr Zeit die Analyse der Messkammer-Probe Pr erfordert, desto größer ist in vielen Fällen das Messrauschen. Eine mögliche Ursache für das Messrauschen ist, dass die elektrische Ladung durch eine zwangsläufig nur näherungsweise Integration über mehrere Messwerte der Stromstärke numerisch ermittelt wird. Das Messrauschen ist in der Regel umso größer, je länger der Zeitraum ist, über den numerisch integriert wird. Ein Grund hierfür ist, dass für die Analyse ein berechneter Nullpunkt verwendet wird und die Berechnung des Nullpunkts zwangsläufig mit Fehlern behaftet ist. Diese Fehler wirken sich häufig umso stärker aus, je länger die Zeitspanne ist.
- Bei einer zu niedrigen Temperatur der Elektroden 20, 21 besteht die Gefahr, dass Feuchtigkeit in der Messkammer 1 oder an einer Fluidführungseinheit, die zur Messkammer 1 führt, kondensiert. Atemalkohol kann kondensieren und / oder sich in der kondensierten Feuchtigkeit lösen. Der elektrochemische Sensor 10 misst dann möglicherweise einen zu geringen Gehalt an Alkohol. Außerdem könnte kondensierte Feuchtigkeit als Bestandteil der Messkammer-Probe Pr in die Messkammer 1 gelangen. Dies kann ebenfalls zu einem falschen Messergebnis führen.

Eine sehr hohe Temperatur der Elektroden 20, 21 kann hingegen den Sensor 10 schädigen. Insbesondere kann ein aus Kunststoff bestehendes Gehäuse 11 beschädigt werden, oder ein Teil des Elektrolyten 28 verdampft, oder schädliche Substanzen setzen sich auf einer Elektrode 20, 21 ab. Außerdem erfordert eine hohe Temperatur mehr elektrische Energie als nötig. Insbesondere dann, wenn das Analysegerät nicht mit einer stationären Spannungsversorgungsnetz verbunden ist, sondern eine eigene Spannungsversorgungseinheit aufweist, sollte daher die Elektroden-Temperatur nicht höher als nötig sein.

Aus folgenden zusätzlichen Gründen sollte die Temperatur der Elektroden 20, 21 nicht zu stark von Messung zu Messung differieren:

- Bei einer vorgegebenen Menge von Atemalkohol in der Messkammer-Probe Pr ist der Messwert, insbesondere das Maß für die elektrische Ladung, umso geringer, je geringer die Temperatur in der Messkammer 1 ist. Je niedriger die Temperatur in der Messkammer 1 ist, desto weniger empfindlich ist also der Sensor 10. Diese Temperaturabhängigkeit erschwert eine Justierung und Kalibrierung des Sensors 10.
- Das Maß für die elektrische Ladung wird in der Regel dadurch gemessen, dass an mehreren Abtast-Zeitpunkten jeweils ein Maß für die Stromstärke gemessen wird und die gemessenen Werte für die Stromstärke aufintegriert werden. Durch dieses Vorgehen tritt zwangsläufig ein Messrauschen auf. Der Einfluss dieses Messrauschens ist umso größer, je länger die Messung dauert, also je mehr Zeit verstreicht, um den Atemalkohol in der Messkammer 1 zu oxidieren. Daher ist der Einfluss des Messrauschens umso größer, je geringer die Temperatur in der Messkammer 1 ist.

In einer Ausgestaltung wird die Temperatur der Messelektrode 20 geregelt, und ein möglicher Temperaturunterschied zwischen den Temperaturen der beiden Elektroden 20, 21 wird vernachlässigt. Eine alternative Ausgestaltung wird weiter unten beschrieben.

In den nachfolgend beschriebenen Figuren wird die gesamte Sensor-Anordnung mit dem Bezugszeichen 100 bezeichnet. Die Sensor-Anordnung 100 umfasst einen elektrochemischen Sensor 10, der so wie mit Bezug auf Figur 2 beschrieben aufgebaut sein kann.

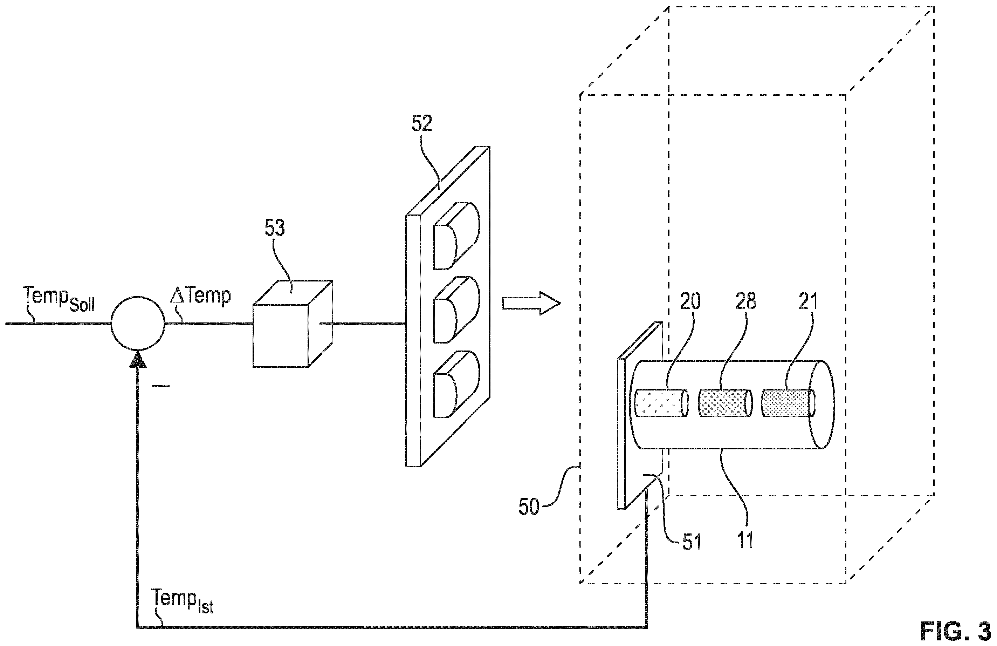

Figur 3 zeigt schematisch einen Regelkreis, um die Temperatur der Messelektrode 20 des Sensors 10 zu regeln. Die Führungsgröße ist im gezeigten Beispiel eine vorgegebene Solltemperatur $Temp_{Soll}$, wobei die Messelektrode 20 konstant diese Solltemperatur $Temp_{Soll}$ aufweisen soll. Die Regelgröße ist die gemessene tatsächliche Temperatur $Temp_{Ist}$ der Messelektrode 20. Die Regelabweichung $Temp_{Soll}$ - $Temp_{Ist}$ wird mit $\Delta Temp$ bezeichnet.

Dieser Regelkreis umfasst folgende Bestandteile:

- eine Regelstrecke 50, die vorliegend die beiden Elektroden 20 und 21 und den Elektrolyten 28 umfasst, wobei die Temperatur der Messelektrode 20 geregelt (auf einem konstanten Wert $Temp_{Soll}$ gehalten) werden soll,
- ein Sensor 51, der die Regelgröße $Temp_{Ist}$ misst und nachfolgend beschrieben wird,
- ein Stellglied 52, welches die Regelgröße $Temp_{Ist}$ beeinflusst, vorliegend eine Heizung, welche die Temperatur der Elektrode 20 zu vergrößern vermag, und

- ein Regler (signalverarbeitendes Steuergerät, control unit) 53, der abhängig von der ermittelten Regelabweichung ∆Temp das Stellglied 52 ansteuert.

Störgrößen sind insbesondere die Umgebungstemperatur und die Temperatur der Atemprobe A und damit der Messkammer-Probe Pr. Auch chemische Reaktionen im Sensor 10 können die Temperatur der Messelektrode 20 beeinflussen und sind eine mögliche weitere Störgröße.

Das Regelungsziel bei dieser Regelung ist, dass die Regelabweichung ∆Temp auf null gebracht werden soll. Weil die Umgebungstemperatur in der Regel geringer oder nur vernachlässigbar größer ist als die Solltemperatur $Temp_{Soll}$, reicht oft eine einseitige Regelung aus, bei der das Stellglied 52 den Wert der Regelgröße zwar vergrößern, aber nicht verkleinern kann. Falls die Sensor-Anordnung 100 bei einer hohen Umgebungstemperatur eingesetzt werden soll, ist es aber auch möglich, dass die Sensor-Anordnung 100 zusätzlich eine ansteuerbare Kühlung aufweist.

Nachfolgend werden jeweils mehrere Ausgestaltungen für den Sensor 51 und für das Stellglied 52 beschrieben. Der Sensor 51 misst die Regelgröße an einer Messposition an oder in einem Messobjekt. Im Ausführungsbeispiel ist das Messobjekt die Messelektrode 20 oder die elektrische Kontaktierung 2 der Messelektrode 20. Als Messobjekt kann auch die Gegenelektrode 21 oder die elektrische Kontaktierung 3 der Gegenelektrode 21 fungieren. Weil die Messposition im oder am Messobjekt 20, 2 ist, wird erreicht, die Regelgröße (die tatsächliche Temperatur $Temp_{Ist}$) mit einen nur geringen Fehler und einer nur sehr geringen Verzögerung (Latenzzeit) zu messen. Würde die Temperatur Temp der Messelektrode 20 an einer Messposition außerhalb des elektrochemischen Sensors 10 gemessen, so kann die gemessene Temperatur an dieser räumlich entfernten Messposition in relevanter Weise von der tatsächlichen Temperatur $Temp_{Ist}$ der Messelektrode 20 zum Mess-Zeitpunkt abweichen.

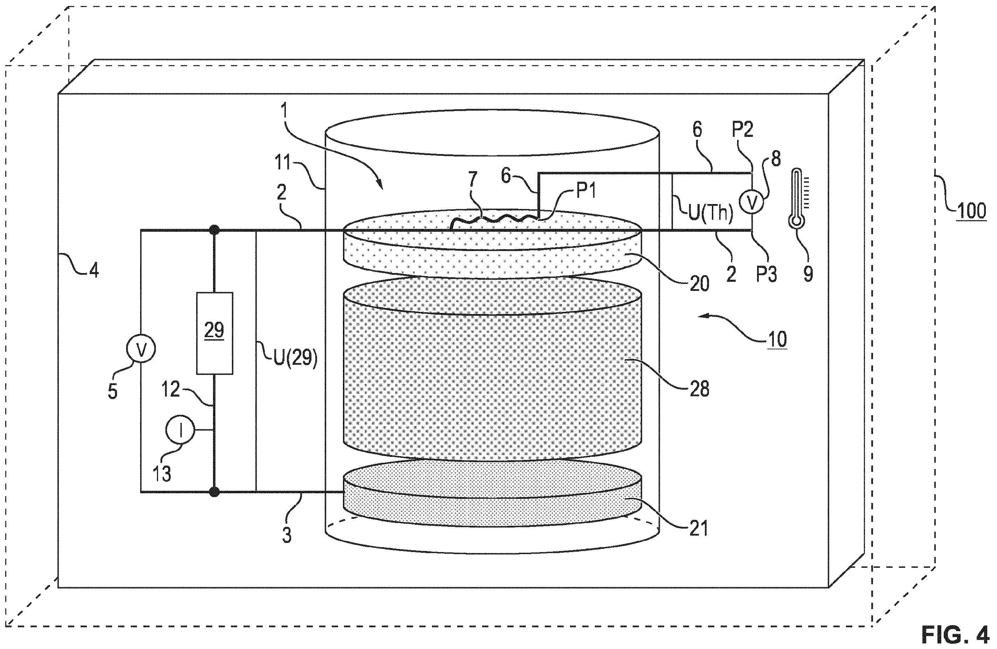

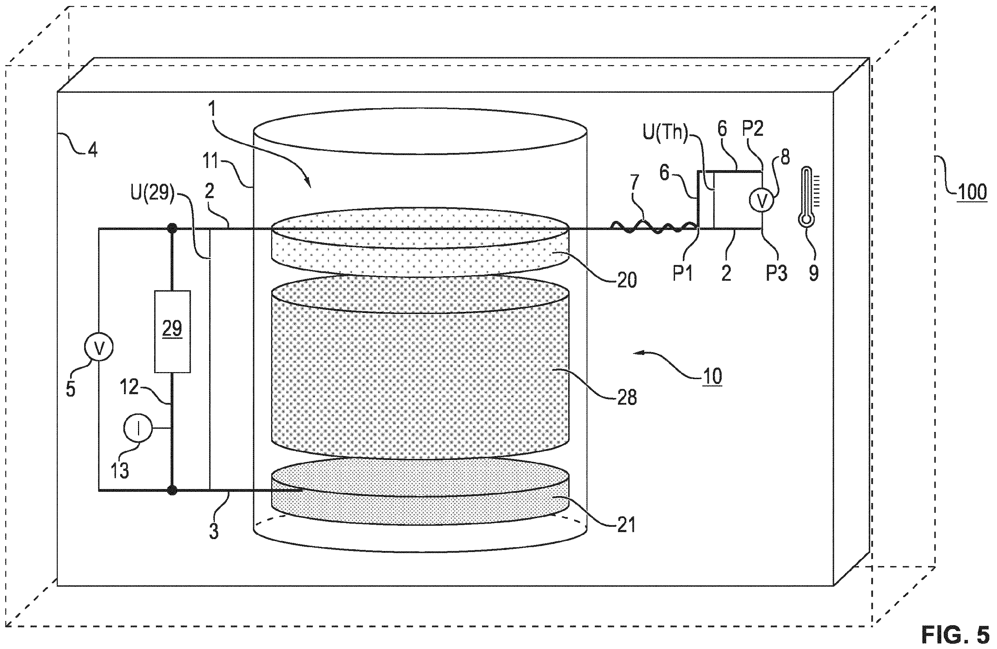

Figur 4 und Figur 5 zeigen schematisch eine Ausgestaltung des TemperaturSensors 51 für die Regelung der Elektroden-Temperatur. Außerdem wird beispielhaft eine Ausgestaltung eines Bereichs der Sensor-Anordnung 100 gezeigt. Zwischen einer Stirnfläche der Messelektrode 20 und einer Stirnfläche der Gegenelektrode 21 befindet sich eine mit dem Elektrolyten 28 getränkte Membrane. Zwischen den beiden Elektroden 20 und 21 ist dank der Membrane 28 ein guter thermischer Kontakt hergestellt. Daher haben die beiden Elektroden 20 und 21 sowie die beiden elektrischen Kontaktierungen 2 und 3 in vielen Fällen annähernd die gleiche Temperatur. An die zylinderförmige Messkammer 1 grenzt eine schematisch gezeigte Platine 4 an.

Zwischen den beiden Drähten 2 und 3, welche die Messelektrode 20 bzw. die Gegenelektrode 21 kontaktieren, ist ein Verbindungsdraht 12 mit einem Messwiderstand 29 angeordnet. Wie bereits dargelegt, bewirkt eine Oxidation von Atemalkohol, dass ein elektrischer Strom durch den Verbindungsdraht 12 fließt. Dieser Strom bewirkt am Messwiderstand 29 einen Spannungsabfall U(29). Ein Spannungs-Sensor 5 auf der Platine 4 misst den Spannungsabfall U(29) am Messwiderstand 29. Dieser Spannungsabfall U(29) ist ein Maß für den Gehalt an Atemalkohol in der Messkammer-Probe Pr und damit der Atemprobe A. In dieser Ausgestaltung ist der Spannungsabfall U(29) eine zusätzliche Detektions-Größe.

In der Ausgestaltung gemäß Figur 4 und Figur 5 misst ein nachfolgend beschriebenes Thermoelement indirekt die übereinstimmende Temperatur der beiden Elektroden 20 und 21 und der beiden elektrischen Kontaktierungen (Platindrähte) 2 und 3. In vielen Fällen differieren diese Temperaturen nur um einen vernachlässigbaren Betrag voneinander. Dieses Thermoelement nutzt den Seebeck-Effekt aus, der weiter oben bereits beschrieben wurde. Nachfolgend werden erfindungsgemäße Ausführungsformen des Thermoelements beschrieben.

In den Ausführungsformen gemäß Figur 4 und Figur 5 umfasst die elektrische Kontaktierung 2 für die Messelektrode 20 einen Platindraht. Die Messelektrode 20 und die elektrische Kontaktierung 2 gehören zum Leiter A. Zu dem Leiter B gehört ein Messelement mit einem anderen Seebeck-Koeffizienten. Dieses Messelement umfasst ein Kontakt-Segment 7 und ein Verbindungs-Segment 6. Das Kontakt-Segment 7 ist beispielsweise aus Gold oder Platin oder Iridium hergestellt und steht in einem thermischen und in einem elektrischen Kontakt mit der Messelektrode 20. Dieser Kontakt zwischen dem Kontakt-Segment 7 der Messelektrode 20 ist beispielsweise in Form einer Wicklung um die Messelektrode 20 oder durch Punktschweißen hergestellt. Dank des thermischen Kontakts weist das Kontakt-Segment 7 annähernd die gleiche Temperatur auf wie die Messelektrode 20. Das Verbindungs-Segment 6 fungiert als ein Messdraht, der das Kontakt-Segment 7 mit der Platine 4 elektrisch und thermisch verbindet.

Gesucht ist die Temperatur Temp(P1) in einem Punkt P1. Diese Temperatur Temp(P1) im Punkt P1 stimmt ausreichend genau mit der gesuchten Temperatur der Messelektrode 20 überein. Der Punkt P1 gehört in der Ausgestaltung gemäß Figur 4 zu dem Bereich, in dem das Kontakt-Segment 7 die Messelektrode 20 kontaktiert. Im gezeigten Beispiel ist das Kontakt-Segment 7 im Punkt P1 mit dem Verbindungs-Segment 6 verbunden. Bevorzugt tritt zwischen dem Punkt P1 und dem Bereich, in dem die elektrische Kontaktierung 2 die Messelektrode 20 berührt, ein Abstand auf.

Der oben erwähnte Leiter A (Messelektrode 20 und elektrische Kontaktierung 2) verbindet den Punkt P1 auf der Messelektrode 20 mit dem Punkt P3 auf der Platine 4. Der Leiter B (Verbindungs-Segment 6) verbindet den Punkt P1 mit dem Punkt P2 auf der Platine 4, vgl. Figur 4 und Figur 5. Bevorzugt weist das Verbindungs-Segment 6 durchgehend den gleichen Seebeck-Koeffizienten k(B) auf. In einer Ausgestaltung weist sogar das gesamte Messelement 6, 7 durchgehend den gleichen Seebeck-Koeffizienten k(B) auf. Letzteres wird bevorzugt dadurch erreicht, dass die beiden Segmente 6 und 7 aus dem gleichen elektrisch leitenden Material hergestellt sind. Möglich ist auch, für die Temperaturmessung einen über die Länge des Verbindungs-Segments 6 oder des Messelements 6, 7 gemittelten Seebeck-Koeffizienten k(B) zu verwenden.

Der Seebeck-Koeffizient k(B) des Verbindungs-Segments 6 unterscheidet sich von dem Seebeck-Koeffizienten k(A) des Leiters A. Der Seebeck-Koeffizient k(A) hängt von dem Seebeck-Koeffizienten der elektrischen Kontaktierung 2 und optional von dem der Messelektrode 20 ab. Beide Seebeck-Koeffizienten k(A) und k(B) sind durch die Konstruktion der Sensor-Anordnung 100 bekannt. Außerdem wird die Voraussetzung verwendet, dass die Seebeck-Koeffizienten k(A) und k(B) - oder wenigstens die Differenz k(A) - k(B) zwischen ihnen - im gesamten Temperaturbereich, in dem die Sensor-Anordnung 100 eingesetzt wird, konstant bleibt.

Möglich ist auch, dass das Kontakt-Segment 7 nicht die Messelektrode 20 kontaktiert, sondern von der Messelektrode 20 beabstandet ist und die elektrische Kontaktierung 2 kontaktiert. Beispielsweise ist das Kontakt-Segment 7 um die elektrische Kontaktierung 2 gewickelt oder verdrillt. Diese abweichende Ausgestaltung wird in Figur 5 gezeigt. Gleiche Bezugszeichen haben die gleiche Bedeutung wie in Figur 4. Der Punkt P1 liegt in dem Bereich, in dem das Kontakt-Segment 7 die elektrische Kontaktierung 2 kontaktiert, und bevorzugt wiederum an der Verbindung zwischen dem Kontakt-Segment 7 und dem Verbindungs-Segment 6.

Möglich ist auch, dass das Verbindungs-Segment 6 die Messelektrode 20 oder die elektrische Kontaktierung 2 in nur einem Punkt P1 kontaktiert. In diesem Falle fungiert dieser idealisiert punktförmige Kontaktbereich als das Kontakt-Segment. Der Seebeck-Koeffizient k(A) ist dann bevorzugt gleich dem Seebeck-Koeffizienten der Messelektrode 20 bzw. der elektrischen Kontaktierung 2.

Das Verbindungs-Segment (der Messdraht 6) sowie die elektrische Kontaktierung 2 sind mit der Platine 4 verbunden. Der Verbindungspunkt zwischen der elektrischen Kontaktierung 2 und der Platine 4 fungiert als der Punkt P2, der Verbindungspunkt zwischen dem Verbindungs-Segment 6 und der Platine 4 als der Punkt P3. Ein Referenz-Temperatur-Sensor 9 auf der Platine 4 misst die Temperatur Temp(P2), also die Temperatur der elektrischen Kontaktierung 2 im Punkt P2, die ausreichend genau mit der Temperatur des Verbindungs-Segments 6 im Punkt P3 übereinstimmt. Möglich ist auch, dass der Referenz-Temperatur-Sensor 9 die Temperatur des Verbindungs-Segments 6 im Punkt P3 misst. Der Referenz-Temperatur-Sensor 9 ist beispielsweise als NTC-Thermistor ausgestaltet (NTC = Negative Temperature Coefficient).

Ein Spannungs-Sensor 8 misst ein Maß für die elektrische Spannung zwischen den Punkten P2 und P3 auf der Platine 4. Diese gemessene Spannung fungiert als die Thermo-Spannung U(Th). Die einzige Unbekannte ist die Temperatur Temp(P1) an der elektrodennahen Messposition P1.

Zu dem Thermoelement der Ausgestaltung gemäß Figur 4 und Figur 5 gehören das Verbindungs-Segment 6, das Kontakt-Segment 7 sowie die Sensoren 8 und 9. Die elektrische Kontaktierung 2 übernimmt zusätzlich eine Funktion im Thermoelement.

In dem Ausführungsbeispiel, das in Figur 4 und Figur 5 gezeigt wird, wird ein Thermoelement verwendet, welches die Temperatur Temp(P1) der Messelektrode 20 misst. In vielen Fällen kann mit ausreichender Genauigkeit angenommen werden, dass die Gegenelektrode 21 stets die gleiche Temperatur wie die Messelektrode 20 aufweist. Möglich ist auch, ein weiteres Thermoelement vorzusehen, welches die Temperatur der Gegenelektrode 21 misst und bevorzugt genauso aufgebaut ist wie das eine Thermoelement in Figur 4.

Ein Vorteil der Ausgestaltungen gemäß Figur 4 und Figur 5 ist, dass weder das Kontakt-Segment 7 noch das Verbindungs-Segment 6 notwendigerweise elektrisch gegen die Messelektrode 20 isoliert zu werden brauchen. Durch das Verbindungs-Segment 6 fließt in der Regel kein nennenswerter Strom. Das Messelement 6, 7 ist bevorzugt aus einem Metall hergestellt, welches chemisch resistent gegen den Elektrolyten 28 ist.

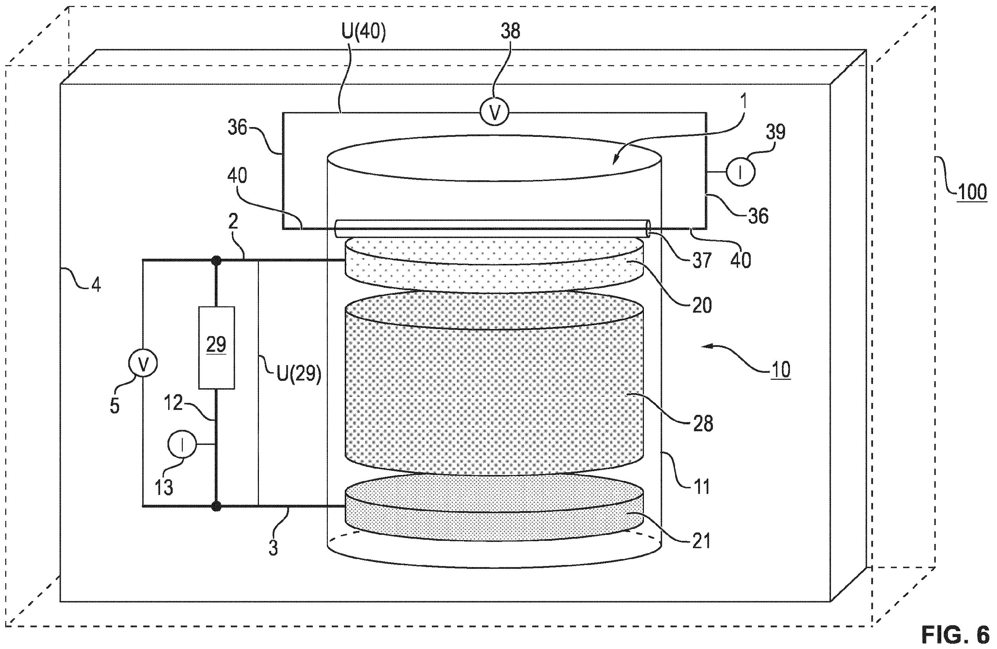

Figur 6 zeigt eine alternative nicht-erfindungsgemäße Ausgestaltung des Sensors 51 für die Regelgröße, wobei im gezeigten Beispiel die Temperatur der Messelektrode 20 die Regelgröße ist. Gleiche Bezugszeichen bezeichnen die gleichen Bauteile wie in Figur 4 und Figur 5.

Ein Kontakt-Segment 40 in Form eines Drahts steht in einem thermischen Kontakt mit einer Stirnfläche der Messelektrode 20. In der gezeigten Realisierungsform ist dies diejenige Stirnfläche, die zur Messkammer 1 zeigt. Möglich ist auch, dass die andere Stirnfläche in thermischem Kontakt mit dem Kontakt-Segment 40 steht. Eine elektrische Isolierung 37 isoliert das Kontakt-Segment 40 elektrisch von der Messelektrode 20. Möglich, aber dank der elektrischen Isolierung 37 in vielen Fällen nicht erforderlich ist, dass das Material des Kontakt-Segments 40 chemisch resistent gegenüber dem Elektrolyten 28 ist. Ein chemisch resistentes Kontakt-Segment 40 wird aber auch dann nicht vom Elektrolyten 28 angegriffen, wenn die elektrische Isolierung 37 eine Fehlstelle (Leck) aufweist. Die elektrische Isolierung 37 weist eine ausreichend hohe thermische Leitfähigkeit auf und umfasst bevorzugt eine isolierende Ummantelung um das Kontakt-Segment 40, beispielsweise aus dünnwandigem Polytetrafluorethylen (PTFE). Die elektrische Isolierung 37 ist ebenfalls chemisch beständig gegen den Elektrolyten 28 und verändert den Elektrolyten 28 nicht.

Das Kontakt-Segment 40 hat die Form eines Drahts, der durch die elektrische Isolierung 37 hindurchgeführt ist. An beiden Enden wird dieses Kontakt-Segment 40 von jeweils einem Verbindungs-Segment 36 kontaktiert. Die Verbindungs-Segmente 36 verbinden die beiden Enden des Kontakt-Segments 40 mit einem Spannungs-Sensor 38. Die beiden Segmente 40, 36 sind Bestandteile eines elektrischen Stromkreises, durch den ein Strom fließt. Bevorzugt steht dieser elektrische Stromkreis in einer elektrischen Verbindung mit einer Spannungsversorgungseinheit des Analysegeräts. Diese Spannungsversorgungseinheit wird in den Figuren nicht gezeigt.

Dank des thermischen Kontakts stimmt die Temperatur des Kontakt-Segments 40 ausreichend genau mit der Temperatur der Messelektrode 20 überein. Bekanntlich hängt der elektrische Widerstand eines elektrisch leitfähigen Materials von der Temperatur des Materials ab, in der Regel dergestalt, dass der elektrische Widerstand umso größer ist, je größer die Temperatur ist (positiver Temperaturkoeffizient). Die Verbindungs-Segmente 36 weisen bei jeder bei einem Einsatz auftretenden Temperatur einen geringeren elektrischen Widerstand auf als das Kontakt-Segment 40. Aus den beiden gerade genannten Gründen ist der elektrische Widerstand des Kontakt-Segments 40 oder auch des ganzen Messelements 40, 36 ein Maß für die Temperatur der Messelektrode 20.

Der Spannungs-Sensor 38 misst ein Maß für den Spannungsabfall U(40) zwischen den beiden Enden des Kontakt-Segments 40. Weil das Kontakt-Segment 40 elektrisch leitend ist, korreliert dieser Spannungsabfall U(40) mit der Temperatur Temp(P1) des Kontakt-Segments 40. Ein Stromstärken-Sensor 39 misst die Stärke I des Stroms, der durch den Stromkreis mit dem Kontakt-Segment 40 und den Verbindungs-Segmenten 36 fließt. Möglich ist, dass die Stärke I des Stroms, durch der durch diesen Stromkreis fließt, mit dem Regelungsziel geregelt wird, dass die Stromstärke konstant bleiben soll. In vielen Fällen ist die gesuchte Temperatur der Messelektrode 20 dann mit ausreichender Genauigkeit proportional zu dem Spannungsabfall U(40). Möglich ist auch, den Spannungsabfall U(40) durch eine Regelung konstant zu halten. Dann ist die Stromstärke I mit ausreichender Genauigkeit proportional zu der gesuchten Temperatur Temp(P1).

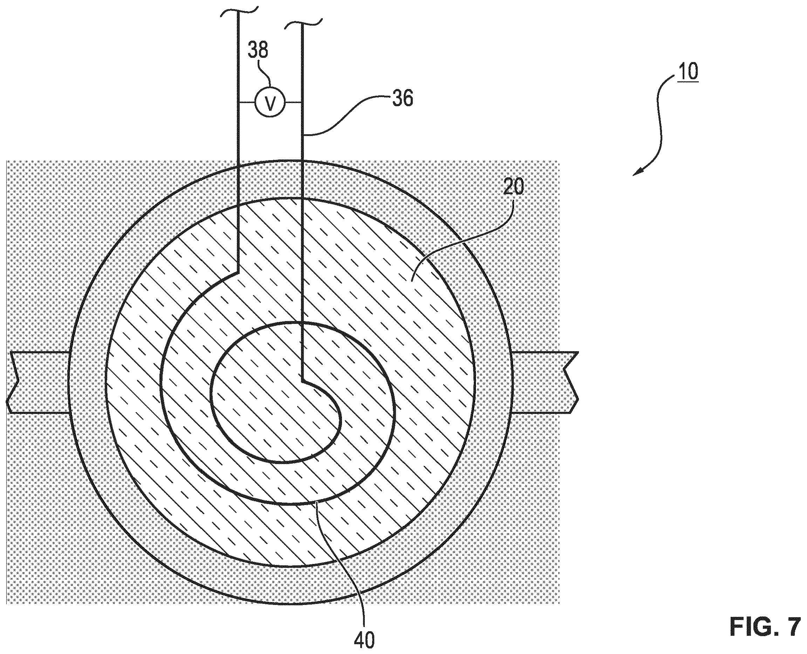

Figur 7 (nicht-erfindungsgemäßes Beispiel) zeigt eine bevorzugte Ausgestaltung des Messelements 40, 36. Die Mittelachse der zylindrischen, insbesondere scheibenförmigen, Messelektrode 20 steht senkrecht auf der Zeichenebene von Figur 7. Figur 7 zeigt, wie das Kontakt-Segment 40 mit einer Stirnfläche der Messelektrode 20 in thermischem Kontakt steht. Um die Strecke desjenigen Abschnitts des Kontakt-Segments 40 zu verlängern, der in thermischem Kontakt mit der Messelektrode 20 steht, weist das Kontakt-Segment 40 bevorzugt mehrere Windungen auf. In Figur 7 wird die elektrische Isolierung 37 nicht gezeigt.

Figur 4 bis Figur 7 zeigen verschiedene Ausgestaltungen, um die aktuelle Temperatur einer Elektrode 20, 21 zu messen. Möglich ist, zwei Ausgestaltungen miteinander zu kombinieren. Insbesondere ist es möglich, dass das Kontakt-Segment 40 sowohl in thermischem Kontakt mit einer Stirnfläche als auch in thermischem Kontakt mit der Mantelfläche der Elektrode 20, 21 steht.

Die nachfolgende Beschreibung bezieht sich auf die Messelektrode 20 als derjenigen Elektrode, deren Temperatur gemessen wird. In einer alternativen Ausgestaltung wird stattdessen oder zusätzlich die aktuelle Temperatur der Gegenelektrode 21 gemessen, beispielsweise ebenfalls durch eine der Ausgestaltungen gemäß Figur 4 bis Figur 7.

Idealerweise haben die Messelektrode 20 und die Gegenelektrode 21 stets dieselbe Temperatur, in der Praxis unterscheiden sie sich in der Regel voneinander. Falls die Temperatur der Messelektrode 20 sich von der Temperatur der Gegenelektrode 21 um mehr als eine vorgegebene Schranke unterscheidet, so detektiert eine signalverarbeitende Auswerteeinheit (nicht gezeigt) der Sensor-Anordnung 100 bevorzugt dieses Ereignis und generiert besonders bevorzugt eine Meldung. Diese Meldung wird in einer von einem Menschen wahrnehmbaren Form ausgegeben.

Bei einem Temperaturunterschied oberhalb der Schranke ist es in vielen Fällen nicht möglich, dass der Sensor 10 den Gehalt an Atemalkohol in eine Atemprobe A zuverlässig misst. Möglich ist, die Sensor-Anordnung 100 erst dann wieder für eine Messung zu verwenden, wenn der Unterschied zwischen den beiden Elektroden-Temperaturen geringer geworden ist und unterhalb der Schranke liegt. In einer anderen Ausgestaltung wird die gemessene Temperatur der Gegenelektrode 21 verwendet, um ein Messergebnis der Messelektrode 20 rechnerisch zu korrigieren.

Der in Figur 3 gezeigte Regelkreis umfasst ein Stellglied 52 in Form einer Heizung, die mindestens die Messelektrode 20 zu beheizen vermag. Weil die Gegenelektrode 21 in einem guten thermischen Kontakt mit der Messelektrode 20 steht, erwärmt die Heizung 52 in der Regel auch die Gegenelektrode 21. Im Folgenden werden zwei bevorzugte Ausgestaltungen dieser Heizung 52 beschrieben.

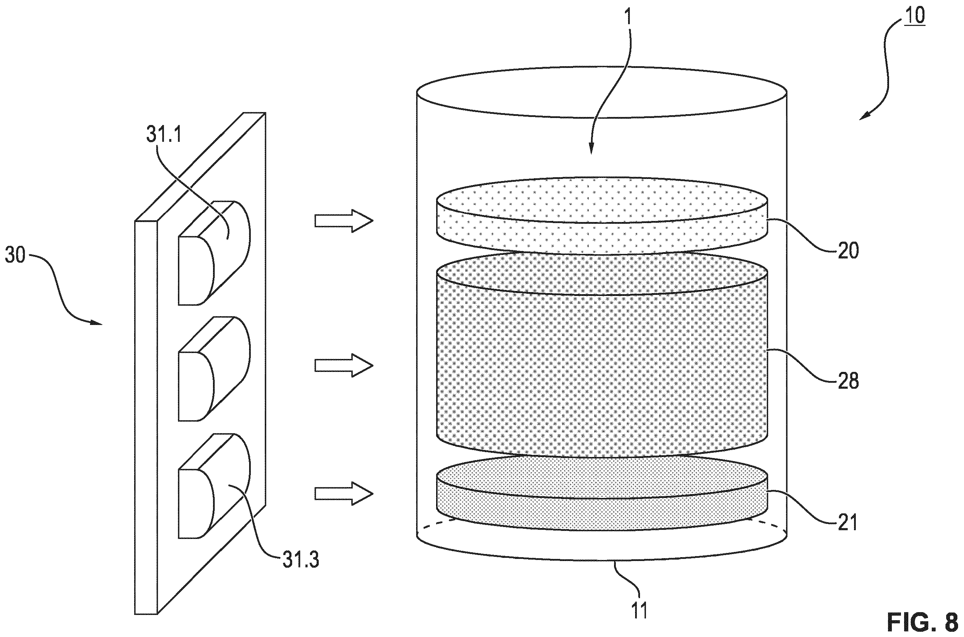

In der Ausgestaltung gemäß Figur 8 umfasst die Heizung 52 eine Strahlungsquelle in Form einer LED-Anordnung 30 mit mindestens einer LED, bevorzugt mehreren LEDs. Beispielhaft sind drei LEDs 31.1 bis 31.3 gezeigt. Eine andere Anzahl ist ebenfalls möglich. Jede LED 31.1 bis 31.3 emittiert elektromagnetische Strahlung. Die Messelektrode 20 und auch die Gegenelektrode 21 sind bevorzugt aus einem dunklen Werkstoff hergestellt und absorbieren daher einen erheblichen Teil der auftreffenden elektromagnetischen Strahlung. Dadurch erwärmen sich die Messelektrode 20 und auch die Gegenelektrode 21. Diese Ausgestaltung ermöglicht es, die Messelektrode 20 und die Gegenelektrode 21 berührungslos zu erwärmen. Die LEDs 31.1 bis 31.3 lassen sich praktisch ohne Zeitverzug einschalten und wieder ausschalten, und bei eingeschalteten LEDs 31.1 bis 31.3 erwärmen sich die Messelektrode 20 und die Gegenelektrode 21 rasch.

In einer Ausgestaltung lassen sich wahlweise alle LEDs 31.1 bis 31.3 oder nur mindestens eine Teilmenge der LEDs einschalten. Dadurch ist es möglich, mindestens zwei unterschiedliche Mengen von Wärmeenergie zu emittieren und dadurch die Messelektrode 20 wahlweise um einen größeren oder um einen kleineren Betrag oder aber schneller oder langsamer zu erwärmen. Bei einer großen Regelabweichung ΔTemp werden bevorzugt alle LEDs 31.1 bis 31.3 eingeschaltet, bei einer kleinen Regelabweichung ΔTemp nur eine Teilmenge der LEDs. Möglich ist auch, die elektrische Spannung, die an den LEDs anliegt, oder die Stärke des durch die LEDs fließenden elektrischen Stroms zu variieren. Dadurch werden die aufgenommene elektrische Leistung und damit auch die emittierte Strahlungsleistung verändert.

In einer weiteren Ausgestaltung werden die LEDs 31.1 bis 31.3 gepulst betrieben. Durch eine Pulsweitenmodulation wird die tatsächlich emittierte Wärmemenge an eine gewünschte zu emittierende Wärmemenge angepasst. Je höher bei gleichbleibender Pulsdauer die Pulsfrequenz oder bei gleichbleibender Pulsfrequenz die Pulsdauer ist, desto höher ist die emittierte Wärmemenge. Möglich ist auch, die Dauer eines Pulses zu vergrößern, um die emittierte Wärmemenge zu steigern. Die Pulsfrequenz wird so festgelegt, dass die thermischen Zeitkonstanten der Elektroden 20, 21 länger sind als die Pulsfrequenz. Dies ist möglich, weil die LEDs 31.1 bis 31.3 eine geringe thermische Masse aufweisen und außerdem schnell eingeschaltet und wieder ausgeschaltet werden können. Die Ausgestaltung mit der Pulsweitenmodulation lässt sich kombinieren mit der Ausgestaltung, dass sich wahlweise alle oder nur ein Teil der LEDs 31.1 bis 31.3 einschalten und ausschalten lassen. Die Ausgestaltung mit der Pulsweitenmodulation ermöglicht aber auch, die Menge der emittierten Wärmeenergie anzupassen und stets alle LEDs 31.1 bis 31.3 einzuschalten und auszuschalten.

In einer bevorzugten Ausgestaltung ist die LED-Anordnung 30 außerhalb des Gehäuses 11 angebracht, beispielsweise außen an einem Gehäuse des Analysegeräts. Diese Ausgestaltung erleichtert es, eine defekte LED 31.1 bis 31.3 oder auch die gesamte LED-Anordnung 30 auszutauschen. Die LED-Anordnung 30 kann so wie in DE 10 2019 003 021 A1 beschrieben ausgestaltet sein.

Bevorzugt emittiert jede LED 31.1 bis 31.3 Strahlung in einem Wellenlängenbereich zwischen 400 und 500 nm. Wellenlängen in diesem Bereich durchdringen viele Arten von Kunststoff, ohne dass der Kunststoff einen erheblichen Teil der transmittierten Strahlung absorbiert, insbesondere dann, wenn der Kunststoff aus einer hellen Farbe ist oder annähernd transparent ist, sodass elektromagnetische Strahlung ohne nennenswerte Absorption durch den Kunststoff transmittiert. Bevorzugt ist das Gehäuse des Analysegeräts mit der Sensor-Anordnung 100 aus einem solchen für elektromagnetische Strahlung transparenten Kunststoff hergestellt. Bevorzugt liegt die maximale Nennleistung der LED-Anordnung 30 zwischen 5W und 7W. Diese Ausgestaltung reicht aus, um die Messelektrode 20 und die Gegenelektrode 21 ausreichend rasch und ausreichend stark zu erwärmen, und verbraucht dennoch nur relativ wenig elektrische Energie.

In einer alternativen Ausgestaltung wird die Messelektrode 20, optional auch die Gegenelektrode 21, von einem Heizdraht erwärmt. Dieser Heizdraht steht in einem thermischen Kontakt mit der zu erwärmenden Elektrode 20, 21 und ist elektrisch von dieser Elektrode 20, 21 isoliert. Besonders bevorzugt wird das Messelement 40, 36 mit dem Kontakt-Segment 40 von Figur 6 und / oder Figur 7 zusätzlich als Heizdraht verwendet. Diese Ausgestaltung spart einen zusätzlichen Heizdraht ein. Der Heizdraht / das Messelement 40, 36 wird erwärmt, um die Messelektrode 20 zu erwärmen, und außerdem wird so wie oben mit Bezug auf Figur 6 beschrieben der elektrische Widerstand des Heizdrahts / Kontakt-Segments 40 gemessen und fungiert als ein Maß für die Temperatur der Messelektrode 20. Weil die am Kontakt-Segment 40 anliegende Spannung U(40) und / oder die Stärke I des durch den Stromkreis mit dem Kontakt-Segment 40 fließenden Stroms gemessen wird, ist es möglich, die Temperatur $Temp_{Ist}$ der Messelektrode 20 mithilfe des Kontakt-Segments 40 zu regeln.

In einer Ausgestaltung ist der Heizdraht / Kontakt-Segment 40 auf eine Kunststofffolie gedruckt. Diese Ausgestaltung ermöglicht in vielen Fällen, den Heizdraht / das Kontakt-Segment 40 rasch und automatisiert zu fertigen.

**Bezugszeichenliste**

| | |
|---|---|
| 1 | Messkammer, nimmt die Messkammer-Probe Pr auf, umgibt den elektrochemischen Sensor 10 |
| 2 | Kontaktierungsdraht aus Platin oder Gold, kontaktiert elektrisch die Messelektrode 20 |
| 3 | Kontaktierungsdraht aus Platin oder Gold, kontaktiert elektrisch die Gegenelektrode 21 |
| 4 | Platine, auf der Spannungs-Sensoren 5 und 6 montiert sind |
| 5 | Spannungs-Sensor, misst den Spannungsabfall U(29) am Messwiderstand 29 |
| 6 | Verbindungs-Segment aus Gold oder Platin, verbindet das Kontakt-Segment 7 mit dem Punkt P2 auf der Platine 4 |
| 7 | Kontakt-Segment aus Gold oder Platin, steht in einem thermischen und optional zusätzlich in einem elektrischen Kontakt mit der Messelektrode 20 oder der elektrischen Kontaktierung 2 |
| 8 | Spannungs-Sensor, misst die Spannung, die aufgrund des Seebeck-Effekts zwischen den beiden Drähten 2 und 6 auftritt |
| 9 | Referenz-Temperatur-Sensor, misst ein Maß für die Temperatur des Drahts 6 an einer Referenz-Position auf der Platine 4 |

(fortgesetzt)

| | |
|---|---|
| 10 | elektrochemischer Sensor in der Messkammer 1, umfasst die Elektroden 20, 21, die elektrischen Kontaktierungen 2 und 3 und den Elektrolyten 28 |
| 11 | Gehäuse des Sensors 10 |
| 12 | Verbindungsdraht zwischen den Kontaktierungsdrähten 2 und 3 |
| 13 | Stromstärken-Sensor, misst die Stärke des durch den Verbindungsdraht 12 fließenden Stroms |
| 20 | Messelektrode des Sensors 10, bevorzugt aus Platin oder Gold |
| 21 | Gegenelektrode des Sensors 10, bevorzugt aus Platin oder Gold |
| 28 | Elektrolyt (Schwefelsäure) im Inneren der Messkammer 1, zwischen den Elektroden 20 und 21 angeordnet |
| 29 | elektrischer Messwiderstand zwischen den beiden Elektroden 20, 21 |
| 30 | LED-Anordnung mit LEDs 31.1, ..., fungiert als Strahlungsquelle und dadurch als Heizung |
| 31.1, ... | LEDs der LED-Anordnung 30, emittieren elektromagnetische Strahlung auf die Elektroden 20, 21 zu |
| 36 | Verbindungs-Segmente, verbinden die beiden Enden des Kontakt-Segments 40 mit dem Spannungs-Sensor 38 |
| 37 | elektrische Isolierung um das Kontakt-Segment 40 |
| 38 | Spannungs-Sensor, misst den Spannungsabfall U(40) zwischen den beiden Enden des Kontakt-Segments 40 |
| 39 | Stromstärken-Sensor, misst die Stärke I des Stroms, der durch das Messelement 40, 36 fließt |
| 40 | Kontakt-Segment, steht in einem thermischen Kontakt mit einer Stirnfläche der Messelektrode 20, durch die Isolierung 37 elektrisch von der Messelektrode 20 isoliert, an seinen beiden Enden mit den beiden Verbindungs-Segmenten 36 verbunden, fungiert in einer Ausgestaltung zusätzlich als Heizelement |
| 50 | Regelstrecke bei der Regelung der Elektroden-Temperatur, umfasst die Elektroden 20, 21 und den Sensor 51 |
| 51 | Temperatur-Sensor, der die Regelgröße (tatsächliche Temperatur der Elektroden 20, 21) misst |
| 52 | Stellglied (Elektrodenheizung), der die Regelgröße verändert |
| 53 | Regler (signalverarbeitendes Steuergerät), welcher das Stellglied 52 ansteuert |
| 100 | erfindungsgemäße Sensor-Anordnung, umfasst den elektrochemischen Sensor 10, den Temperatur-Sensor, die Heizung, die optionale Kühlung und das Steuergerät 53, gehört zu einem Analysegerät |
| A | elektrischer Leiter vom Punkt P1 zum Punkt P3 |
| Ap | vom Probanden abgegebene Atemprobe, umfasst die Messkammer-Probe Pr |
| B | elektrischer Leiter vom Punkt P1 zum Punkt P2 |
| Ö.a | austrittseitige Öffnung im Gehäuse 11, durch welche hindurch die Messkammer-Probe Pr aus der Messkammer 1 heraus fließt |
| Ö.e | eintrittsseitige Öffnung im Gehäuse 11, durch welche hindurch die Messkammer-Probe Pr in die Messkammer 1 hinein fließt |
| P1 | Punkt des Kontakt-Segments 7, 40, in denen die beiden Leiter A und B beginnen |
| P2, P3 | Punkte auf der Platine 4, wobei zwischen diesen Punkten die Thermospannung U(Th) auftritt |
| Pr | Messkammer-Probe, das ist derjenige Teil der vom Probanden abgegebene Atemprobe A, die in die Messkammer 1 gelangt, stammt idealerweise aus der Lunge des Probanden, wird auf Atemalkohol untersucht |
| $\Delta$Temp | Regelabweichung der Regelstrecke 50, gleich $Temp_{Soll}$ - $Temp_{Ist}$ |
| $Temp_{Soll}$ | vorgegebene Solltemperatur der Regelstrecke 50 |
| $Temp_{Ist}$ | gemessene tatsächliche Temperatur der Regelstrecke 50 |

(fortgesetzt)

| U(29) | Spannungsabfall am Messwiderstand 29, vom Spannungs-Sensor 5 gemessen |
|---|---|
| U(40) | Spannungsabfall zwischen den beiden Enden des Kontakt-Segments 40, vom Spannungs-Sensor 38 gemessen |
| U(Th) | Thermo-Spannung zwischen den Drähten 2 und 6 auf der Platine 4 und damit zwischen den Punkten P2 und P3, vom Spannungs-Sensor 8 gemessen |

**Patentansprüche**

1. Sensor-Anordnung (100) zum Analysieren eines Gases (Pr) auf mindestens einen vorgegebenen Gas-Bestandteil,

wobei die Sensor-Anordnung (100)

- einen elektrochemischen Sensor (10) und
- eine Temperatur-Sensoreinheit (6, 7, 8, 9, 36, 37, 38, 39, 40, 51) umfasst,

wobei der elektrochemische Sensor (10)

- eine Messelektrode (20),
- eine Gegenelektrode (21),
- eine elektrische Kontaktierung (2) für die Messelektrode (20),
- eine elektrische Kontaktierung (3) für die Gegenelektrode (21) und
- einen Elektrolyten (28) zwischen der Messelektrode (20) und der Gegenelektrode (21)

umfasst,
wobei die Temperatur-Sensoreinheit (6, 7, 8, 9, 36, 37, 38, 39, 40, 51) ein elektrisch leitendes Messelement (6, 7, 36, 37, 40) umfasst,
wobei die Sensor-Anordnung (100) dazu ausgestaltet ist, eine Detektions-Größe des elektrochemischen Sensors (10) zu messen,
wobei die messbare Detektions-Größe mit dem Vorhandensein und / oder der Konzentration des oder mindestens eines Gas-Bestandteils korreliert,
wobei das Messelement (6, 7, 36, 37, 40) ein Kontakt-Segment (7, 40) und ein Verbindungs-Segment (6, 36) umfasst,
wobei das Kontakt-Segment (7, 40) dergestalt in einem flächigen Kontakt mit einem Messobjekt (20, 21, 2, 3) des elektrochemische Sensors (10) steht, dass ein thermischer Kontakt zwischen dem Kontakt-Segment (7, 40) und dem Messobjekt (20, 21, 2, 3) hergestellt ist,
wobei das Messobjekt (20, 21, 2, 3) die Messelektrode (20), die Gegenelektrode (21), die elektrische Kontaktierung (2) für die Messelektrode (20) oder die elektrische Kontaktierung (3) für die Gegenelektrode (21) ist, und
wobei die Temperatur-Sensoreinheit (6, 7, 8, 9, 36, 37, 38, 39, 40, 51) dazu ausgestaltet ist, abhängig von einer gemessenen mit der Kontakt-Segment-Temperatur korrelierenden Größe wenigstens näherungsweise die Temperatur der Messelektrode (20) und / oder die Temperatur der Gegenelektrode (21) zu ermitteln,
**dadurch gekennzeichnet, dass**
die Temperatur-Sensoreinheit (6, 7, 8, 9, 36, 37, 38, 39, 40, 51) zusätzlich einen Temperatur-Sensor (9, 38, 39) und einen Spannungs-Sensor (8) umfasst,
wobei das Kontakt-Segment (7, 40) mit dem Messobjekt (20, 21, 2, 3) zusätzlich elektrisch leitend verbunden ist,
wobei das Verbindungs-Segment (6, 36) das Kontakt-Segment (7, 40) mit dem Temperatur-Sensor (9, 38, 39) elektrisch und / oder thermisch verbindet,
wobei das Verbindungs-Segment (6, 36) einen anderen Seebeck-Koeffizienten als das Messobjekt (20, 21, 2, 3) aufweist,
wobei der Spannungs-Sensor (8) dazu ausgestaltet ist, ein Maß für eine Thermo-Spannung [U(Th)], die zwischen

- dem Verbindungs-Segment (6, 36) einerseits und
- dem Messobjekt (20, 21) oder der elektrischen Kontaktierung (2, 3) für das Messobjekt (20, 21) andererseits

auftritt, zu messen,

wobei der Temperatur-Sensor (9, 38, 39) dazu ausgestaltet ist, an einer Messposition (P2, P3) als die Größe, die mit der Temperatur des Kontakt-Segments (7, 40) korreliert, eine Größe, die mit der Temperatur des Verbindungs-Segments (6, 36) korreliert, zu messen,

wobei die Messposition (P2, P3) räumlich von beiden Elektroden (20, 21) und von beiden elektrischen Kontaktierungen (2, 3) beabstandet ist, und

wobei die Temperatur-Sensoreinheit (6, 7, 8, 9, 36, 37, 38, 39, 40 dazu ausgestaltet ist, abhängig von

- der gemessenen Thermo-Spannung [U(Th)] und
- der gemessenen Größe, die mit der Temperatur des Verbindungs-Segments (6, 36) korreliert,

die Temperatur ($Temp_{Ist}$) des Messobjekts (20, 21) zu ermitteln.

2. Sensor-Anordnung (100) nach Anspruch 1,
**dadurch gekennzeichnet, dass**

das Messobjekt die Messelektrode (20) oder die Gegenelektrode (21) ist,

das Verbindungs-Segment (6, 36) einen anderen Seebeck-Koeffizienten als die elektrische Kontaktierung (2, 3) des Messobjekts (20, 21) aufweist und

der Spannungs-Sensor (8) dazu ausgestaltet ist, ein Maß für eine Thermo-Spannung [U(Th)], die zwischen

- dem Verbindungs-Segment (6, 36) einerseits und
- der elektrischen Kontaktierung (2, 3) des Messobjekts (20, 21) andererseits

auftritt, zu messen.

3. Sensor-Anordnung (100) nach Anspruch 2,
**dadurch gekennzeichnet, dass**

der Temperatur-Sensor (9, 38, 39) dazu ausgestaltet ist,

- die Temperatur der elektrischen Kontaktierung (2, 3) des Messobjekts (20, 21) oder
- eine Größe, die mit der Temperatur der elektrischen Kontaktierung (2, 3) des Messobjekts (20, 21) korreliert,

zu messen.

4. Sensor-Anordnung (100) nach einem der vorhergehenden Ansprüche,
**dadurch gekennzeichnet, dass**

die Sensor-Anordnung (100) zusätzlich

- eine ansteuerbare Heizung (30, 36, 37, 38, 39, 52) und
- ein signalverarbeitendes Steuergerät (53)

umfasst,

wobei die Heizung (30, 36, 37, 38, 39, 52) dazu ausgestaltet ist, die Messelektrode (20) und / oder die Gegenelektrode (21) zu erwärmen, und

wobei das Steuergerät (53) dazu ausgestaltet ist,

- die tatsächliche Temperatur ($Temp_{Ist}$) der Messelektrode (20) und / oder der Gegenelektrode (21) mit dem Regelungsziel zu regeln,

dass die geregelte Temperatur ($Temp_{Ist}$) in einem vorgegebenen Temperaturbereich ($Temp_{Soll}$) bleibt, und

- für die Regelung der tatsächlichen Temperatur ($Temp_{Ist}$) die Heizung (30, 36, 37, 38, 39, 52) abhängig von einem Signal der Temperatur-Sensoreinheit (6, 7, 8, 9, 36, 37, 38, 39, 40, 51) anzusteuern.

5. Sensor-Anordnung (100) nach Anspruch 4,
**dadurch gekennzeichnet, dass**

die Heizung (30, 36, 37, 38, 39, 52) eine ansteuerbare Strahlungsquelle (30) umfasst,

wobei die Strahlungsquelle (30) dazu ausgestaltet ist, eine elektromagnetische Strahlung in Richtung der Messelektrode (20) und / oder der Gegenelektrode (21) zu emittieren, und

wobei das Steuergerät (53) dazu ausgestaltet ist, abhängig von einem Signal der Temperatur-Sensoreinheit (6, 7, 8, 9, 36, 37, 38, 39, 40, 51) zu bewirken, dass die Intensität und / oder die Energie der von der Strahlungsquelle (30) emittierten Strahlung auf einen Wert eingestellt wird.

6. Sensor-Anordnung (100) nach einem der Ansprüche 4 oder 5,
**dadurch gekennzeichnet, dass**

das Steuergerät dazu ausgestaltet ist, abhängig von einem Signal der Temperatur-Sensoreinheit (6, 7, 8, 9, 36, 37, 38, 39, 40, 51) einen Sollwert für die elektrische Spannung, die an der Heizung (30, 36, 37, 38, 39, 52) anliegen soll, zu berechnen und

die Sensor-Anordnung (100) (100) dazu ausgestaltet ist, die tatsächlich an der Heizung (30, 36, 37, 38, 39, 52) anliegende elektrische Spannung auf den Sollwert einzustellen.

7. Sensor-Anordnung (100) nach einem der Ansprüche 4 bis 6,
**dadurch gekennzeichnet, dass**

die Sensor-Anordnung (100) zusätzlich eine ansteuerbare Kühlung umfasst,

wobei die Kühlung dazu ausgestaltet ist, die Messelektrode (20) und / oder die Gegenelektrode (21) zu kühlen, und

wobei das Steuergerät (53) dazu ausgestaltet ist,

für die Regelung der Temperatur ($Temp_{Ist}$) zusätzlich abhängig von einem Signal der Temperatur-Sensoreinheit (6, 7, 8, 9, 36, 37, 38, 39, 40, 51) die Kühlung anzusteuern.

8. Analysegerät umfassend

- eine Sensor-Anordnung (100) nach einem der vorhergehenden Ansprüche und
- eine Eingabeeinheit,

wobei die Eingabeeinheit dazu ausgestaltet ist, eine Atemprobe (Ap) von einem Probanden aufzunehmen, und

wobei das Analysegerät dazu ausgestaltet ist, wenigstens einen Teil der von der Eingabeeinheit aufgenommenen Atemprobe (Ap) zum elektrochemischen Sensor (10) zu leiten.

9. Verfahren zum Analysieren eines Gases (Pr) auf mindestens einen vorgegebenen Gas-Bestandteil

unter Verwendung einer Sensor-Anordnung (100), die

- einen elektrochemischen Sensor (10) und
- eine Temperatur-Sensoreinheit (6, 7, 8, 9, 36, 37, 38, 39, 40, 51) umfasst,

wobei der elektrochemische Sensor (10)

- eine Messelektrode (20),
- eine Gegenelektrode (21),
- eine elektrische Kontaktierung (2) für die Messelektrode (20),
- eine elektrische Kontaktierung (3) für die Gegenelektrode (21) und
- einen Elektrolyten (28) zwischen der Messelektrode (20) und der Gegenelektrode (21)

umfasst,

wobei die Temperatur-Sensoreinheit (6, 7, 8, 9, 36, 37, 38, 39, 40, 51) ein elektrisch leitendes Messelement (6, 7, 36, 37, 40) umfasst,

wobei das Messelement (6, 7, 36, 37, 40) ein Kontakt-Segment (7, 40) und ein Verbindungs-Segment (6, 36) umfasst,

wobei das Kontakt-Segment (7, 40) dergestalt in einem flächigen Kontakt mit einem Messobjekt (20, 21, 2, 3) des elektrochemische Sensors (10) steht, dass ein thermischer Kontakt zwischen dem Kontakt-Segment (7, 40) und dem Messobjekt (20, 21, 2, 3) hergestellt ist,

wobei das Messobjekt (20, 21, 2, 3) die Messelektrode (20), die Gegenelektrode (21), die elektrische Kontaktierung (2) für die Messelektrode (20) oder die elektrische Kontaktierung (3) für die Gegenelektrode (21) ist,

wobei das Verbindungs-Segment (6, 36) das Kontakt-Segment (7, 40) mit dem Temperatur-Sensor (9, 38, 39) elektrisch und / oder thermisch verbindet,

wobei das Verfahren die automatisch durchgeführten Schritte umfasst, dass

- eine Detektions-Größe gemessen wird,

wobei die gemessene Detektions-Größe mit dem Vorhandensein und / oder der Konzentration des oder mindestens eines Gas-Bestandteils korreliert,

- eine Größe, die mit der Temperatur des Kontakt-Segments (7, 40) korreliert, gemessen wird und

- die Temperatur-Sensoreinheit (6, 7, 8, 9, 36, 37, 38, 39, 40, 51) abhängig von der gemessenen mit der Kontakt-Segment-Temperatur korrelierenden Größe wenigstens näherungsweise die Temperatur der Messelektrode (20) und / oder die Temperatur der Gegenelektrode (21) ermittelt,

**dadurch gekennzeichnet, dass**

die Temperatur-Sensoreinheit (6, 7, 8, 9, 36, 37, 38, 39, 40, 51) zusätzlich einen Temperatur-Sensor (9, 38, 39) und einen Spannungs-Sensor (8) umfasst,

wobei das Kontakt-Segment (7, 40) mit dem Messobjekt (20, 21, 2, 3) zusätzlich elektrisch leitend verbunden ist,

wobei das Verbindungs-Segment (6, 36) das Kontakt-Segment (7, 40) mit dem Temperatur-Sensor (9, 38, 39) elektrisch und / oder thermisch verbindet, wobei das Verbindungs-Segment (6, 36) einen anderen Seebeck-Koeffizienten als das Messobjekt (20, 21, 2, 3) aufweist,

wobei das Verfahren zusätzlich die automatisch durchgeführten Schritte umfasst, dass

der Temperatur-Sensor (9, 38, 39) an einer Messposition (P2, P3) als die Größe, die mit der Temperatur des Kontakt-Segments (7, 40) korreliert, eine Größe, die mit der Temperatur des Verbindungs-Segments (6, 36) korreliert, misst,

wobei die Messposition (P2, P3) räumlich von beiden Elektroden (20, 21) und von beiden elektrischen Kontaktierungen (2, 3) beabstandet ist,

der Spannungs-Sensor (8) ein Maß für eine Thermo-Spannung [U(Th)], die zwischen

- dem Verbindungs-Segment (6, 36) einerseits und

- dem Messobjekt (20, 21) oder der elektrischen Kontaktierung (2, 3) für das Messobjekt (20, 21) andererseits

auftritt, misst, und

die Temperatur-Sensoreinheit (6, 7, 8, 9, 36, 37, 38, 39, 40, 51) die Temperatur (Temp$_{Ist}$) des Messobjekts (20, 21) abhängig von

- der gemessenen Thermo-Spannung [U(Th)] und

- der gemessenen Größe, die mit der Temperatur des Verbindungs-Segments (6, 36) korreliert,

ermittelt.

10. Verfahren nach Anspruch 9,
**dadurch gekennzeichnet, dass**

die Sensor-Anordnung (100) zusätzlich

- eine ansteuerbare Heizung (30, 36, 37, 38, 39, 52) und

- ein signalverarbeitendes Steuergerät (53)

umfasst und

das Verfahren zusätzlich den automatisch durchgeführten Schritt umfasst, dass

das Steuergerät (53) die tatsächliche Temperatur (Temp$_{Ist}$) der Messelektrode (20) und / oder der Gegenelektrode (21) mit dem Regelungsziel regelt, dass diese tatsächliche Temperatur (Temp$_{Ist}$) in einem vorgegebenen Temperaturbereich (Temp$_{Soll}$) bleibt,

wobei dann, wenn die ermittelte tatsächliche Temperatur (Temp$_{Ist}$) unterhalb des Temperaturbereichs (Temp$_{Soll}$) liegt,

- das Steuergerät (53) die Heizung (30, 36, 37, 38, 39, 52) aktiviert,
- die aktivierte Heizung (30, 36, 37, 38, 39, 52) die Messelektrode (20) und / oder die Gegenelektrode (21) erwärmt und
- das Steuergerät (53) später die Heizung (30, 36, 37, 38, 39, 52) wieder deaktiviert.

**Claims**

1. Sensor arrangement (100) for analyzing a gas (Pr) for at least one predetermined gas component,

   the sensor arrangement (100) comprising

   - an electrochemical sensor (10) and
   - a temperature sensor unit (6, 7, 8, 9, 36, 37, 38, 39, 40, 51),

   the electrochemical sensor (10) comprising

   - a measurement electrode (20),
   - a counter electrode (21),
   - an electrical contact (2) for the measurement electrode (20),
   - an electrical contact (3) for the counter electrode (21), and
   - an electrolyte (28) between the measurement electrode (20) and the counter electrode (21),

   the temperature sensor unit (6, 7, 8, 9, 36, 37, 38, 39, 40, 51) comprising an electrically conductive measurement element (6, 7, 36, 37, 40),
   the sensor arrangement (100) being configured to measure a detection variable of the electrochemical sensor (10),
   the measurable detection variable correlating with the presence and/or concentration of the or at least one gas component,
   the measurement element (6, 7, 36, 37, 40) comprising a contact segment (7, 40) and a connecting segment (6, 36),
   the contact segment (7, 40) being in planar contact with a measurement object (20, 21, 2, 3) of the electrochemical sensor (10) in such a way that thermal contact is established between the contact segment (7, 40) and the measurement object (20, 21, 2, 3),
   the measurement object (20, 21, 2, 3) being the measurement electrode (20), the counter electrode (21), the electrical contact (2) for the measurement electrode (20), or the electrical contact (3) for the counter electrode (21), and
   the temperature sensor unit (6, 7, 8, 9, 36, 37, 38, 39, 40, 51) being configured to determine, at least approximately, the temperature of the measurement electrode (20) and/or the temperature of the counter electrode (21) depending on a measured variable correlating with the contact segment temperature,
   **characterized in that**
   the temperature sensor unit (6, 7, 8, 9, 36, 37, 38, 39, 40, 51) additionally comprises a temperature sensor (9, 38, 39) and a voltage sensor (8),
   the contact segment (7, 40) additionally being electrically conductively connected to the measurement object (20, 21, 2, 3),
   the connecting segment (6, 36) electrically and/or thermally connecting the contact segment (7, 40) to the temperature sensor (9, 38, 39),
   the connecting segment (6, 36) having a different Seebeck coefficient to the measurement object (20, 21, 2, 3),
   the voltage sensor (8) being configured to measure an amount of a thermovoltage [U(Th)] which occurs between

   - the connecting segment (6, 36) and
   - the measurement object (20, 21) or the electrical contact (2, 3) for the measurement object (20, 21),

   the temperature sensor (9, 38, 39) being configured to measure, at a measuring position (P2, P3), a variable that correlates with the temperature of the connecting segment (6, 36) as the variable that correlates with the temperature of the contact segment (7, 40),
   the measuring position (P2, P3) being spatially at a distance from the two electrodes (20, 21) and from the two electrical contacts (2, 3), and

the temperature sensor unit (6, 7, 8, 9, 36, 37, 38, 39, 40) being configured,
depending on

- the measured thermovoltage [U(Th)] and
- the measured variable that correlates with the temperature of the connecting segment (6, 36),

to determine the temperature ($Temp_{Ist}$) of the measurement object (20, 21).

2. Sensor arrangement (100) according to claim 1,
**characterized in that**

the measurement object is the measurement electrode (20) or the counter electrode (21),
the connecting segment (6, 36) has a different Seebeck coefficient to the electrical contact (2, 3) of the measurement object (20, 21), and
the voltage sensor (8) is configured to measure an amount of a thermovoltage [U(Th)] which occurs between

- the connecting segment (6, 36) and
- the electrical contact (2, 3) of the measurement object (20, 21).

3. Sensor arrangement (100) according to claim 2,
**characterized in that**
the temperature sensor (9, 38, 39) is configured to measure

- the temperature of the electrical contact (2, 3) of the measurement object (20, 21) or
- a variable that correlates with the temperature of the electrical contact (2, 3) of the measurement object (20, 21).

4. Sensor arrangement (100) according to any of the preceding claims, **characterized in that**

the sensor arrangement (100) additionally comprises

- a controllable heating system (30, 36, 37, 38, 39, 52) and
- a signal-processing control unit (53),

the heating system (30, 36, 37, 38, 39, 52) being configured to heat the measurement electrode (20) and/or the counter electrode (21), and
the control unit (53) being configured

- to control the actual temperature ($Temp_{Ist}$) of the measurement electrode (20) and/or the counter electrode (21) with the control objective

that the controlled temperature ($Temp_{Ist}$) remains in a predetermined temperature range ($Temp_{Soll}$), and

- to control the heating system (30, 36, 37, 38, 39, 52) depending on a signal from the temperature sensor unit (6, 7, 8, 9, 36, 37, 38, 39, 40, 51) in order to control the actual temperature ($Temp_{Ist}$).

5. Sensor arrangement (100) according to claim 4,
**characterized in that**

the heating system (30, 36, 37, 38, 39, 52) comprises a controllable radiation source (30),
the radiation source (30) being configured to emit electromagnetic radiation in the direction of the measurement electrode (20) and/or the counter electrode (21), and
the control unit (53) being configured, depending on a signal from the temperature sensor unit (6, 7, 8, 9, 36, 37, 38, 39, 40, 51), to cause the intensity and/or the energy of the radiation emitted by the radiation source (30) to be adjusted to a value.

6. Sensor arrangement (100) according to either claim 4 or claim 5,
**characterized in that**

EP 4 303 577 B1

the control unit is configured to calculate a target value for the electrical voltage to be applied to the heating system (30, 36, 37, 38, 39, 52) depending on a signal from the temperature sensor unit (6, 7, 8, 9, 36, 37, 38, 39, 40, 51), and

the sensor arrangement (100) (100) is configured to adjust the electrical voltage actually applied to the heating system (30, 36, 37, 38, 39, 52) to the target value.

7. Sensor arrangement (100) according to any of claims 4 to 6,
   **characterized in that**

   the sensor arrangement (100) additionally comprises a controllable cooling system,
   the cooling system being configured to cool the measurement electrode (20) and/or the counter electrode (21), and
   the control unit (53) being configured
   to additionally control the cooling system depending on a signal from the temperature sensor unit (6, 7, 8, 9, 36, 37, 38, 39, 40, 51) in order to control the temperature ($Temp_{Ist}$).

8. Analysis device comprising

   - a sensor arrangement (100) according to any of the preceding claims, and
   - an input unit,

     wherein the input unit is configured to receive a breath sample (Ap) from a test subject, and
     wherein the analysis device is configured to direct at least a portion of the breath sample (Ap) received by the input unit to the electrochemical sensor (10).

9. Method for analyzing a gas (Pr) for at least one predetermined gas component

   using a sensor arrangement (100) which comprises

     - an electrochemical sensor (10) and
     - a temperature sensor unit (6, 7, 8, 9, 36, 37, 38, 39, 40, 51),

   the electrochemical sensor (10) comprising

     - a measurement electrode (20),
     - a counter electrode (21),
     - an electrical contact (2) for the measurement electrode (20),
     - an electrical contact (3) for the counter electrode (21), and
     - an electrolyte (28) between the measurement electrode (20) and the counter electrode (21),

   the temperature sensor unit (6, 7, 8, 9, 36, 37, 38, 39, 40, 51) comprising an electrically conductive measurement element (6, 7, 36, 37, 40),
   the measurement element (6, 7, 36, 37, 40) comprising a contact segment (7, 40) and a connecting segment (6, 36),
   the contact segment (7, 40) being in planar contact with a measurement object (20, 21, 2, 3) of the electrochemical sensor (10) in such a way that thermal contact is established between the contact segment (7, 40) and the measurement object (20, 21, 2, 3),
   the measurement object (20, 21, 2, 3) being the measurement electrode (20), the counter electrode (21), the electrical contact (2) for the measurement electrode (20) or the electrical contact (3) for the counter electrode (21),
   the connecting segment (6, 36) electrically and/or thermally connecting the contact segment (7, 40) to the temperature sensor (9, 38, 39),
   the method comprising the following automatically performed steps:

     - a detection variable is measured,

   the measured detection variable correlating with the presence and/or concentration of the or at least one gas component,

- a variable that correlates with the temperature of the contact segment (7, 40) is measured, and
- the temperature sensor unit (6, 7, 8, 9, 36, 37, 38, 39, 40, 51) determines, at least approximately, the temperature of the measurement electrode (20) and/or the temperature of the counter electrode (21) depending on the measured variable correlating with the contact segment temperature,

**characterized in that**
the temperature sensor unit (6, 7, 8, 9, 36, 37, 38, 39, 40, 51) additionally comprises a temperature sensor (9, 38, 39) and a voltage sensor (8),
the contact segment (7, 40) additionally being electrically conductively connected to the measurement object (20, 21, 2, 3),
the connecting segment (6, 36) electrically and/or thermally connecting the contact segment (7, 40) to the temperature sensor (9, 38, 39),
the connecting segment (6, 36) having a different Seebeck coefficient to the measurement object (20, 21, 2, 3),
the method additionally comprising the following automatically performed steps:

the temperature sensor (9, 38, 39) measures, at a measuring position (P2, P3), a variable that correlates with the temperature of the connecting segment (6, 36) as the variable that correlates with the temperature of the contact segment (7, 40),
the measuring position (P2, P3) being spatially at a distance from the two electrodes (20, 21) and from the two electrical contacts (2, 3),
the voltage sensor (8) measures an amount of a thermovoltage [U(Th)] which occurs between

- the connecting segment (6, 36) and
- the measurement object (20, 21) or the electrical contact (2, 3) for the measurement object (20, 21),

and
the temperature sensor unit (6, 7, 8, 9, 36, 37, 38, 39, 40, 51) determines the temperature ($Temp_{Ist}$) of the measurement object (20, 21) depending on

- the measured thermovoltage [U(Th)] and
- the measured variable that correlates with the temperature of the connecting segment (6, 36).

**10.** Method according to claim 9,
**characterized in that**

the sensor arrangement (100) additionally comprises

- a controllable heating system (30, 36, 37, 38, 39, 52) and
- a signal-processing control unit (53),

and
the method additionally comprises the following automatically performed step:

the control unit (53) controls the actual temperature ($Temp_{Ist}$) of the measurement electrode (20) and/or the counter electrode (21) with the control objective that this actual temperature ($Temp_{Ist}$) remains in a predetermined temperature range ($Temp_{Soll}$),
if the determined actual temperature ($Temp_{Ist}$) is below the temperature range ($Temp_{Soll}$),

- the control unit (53) activating the heating system (30, 36, 37, 38, 39, 52),
- the activated heating system (30, 36, 37, 38, 39, 52) heating the measurement electrode (20) and/or the counter electrode (21), and
- the control unit (53) later deactivating the heating system (30, 36, 37, 38, 39, 52) again.

**Revendications**

**1.** Agencement de capteurs (100) permettant d'analyser un gaz (Pr) en ce qui concerne au moins un composant gazeux prédéterminé,

dans lequel l'agencement de capteurs (100) comprend

- un capteur électrochimique (10) et
- une unité à capteurs de température (6, 7, 8, 9, 36, 37, 38, 39, 40, 51),

dans lequel le capteur électrochimique (10) comprend

- une électrode de mesure (20),
- une contre-électrode (21),
- un moyen de mise en contact électrique (2) pour l'électrode de mesure (20),
- un moyen de mise en contact électrique (3) pour la contre-électrode (21) et
- un électrolyte (28) entre l'électrode de mesure (20) et la contre-électrode (21),

dans lequel l'unité à capteurs de température (6, 7, 8, 9, 36, 37, 38, 39, 40, 51) comprend un élément de mesure (6, 7, 36, 37, 40) électriquement conducteur,
dans lequel l'agencement de capteurs (100) est configuré pour mesurer une grandeur de détection du capteur électrochimique (10),
dans lequel la grandeur de détection mesurable est corrélée avec la présence et/ou la concentration du composant gazeux ou d'au moins un composant gazeux,
dans lequel l'élément de mesure (6, 7, 36, 37, 40) comprend un segment de contact (7, 40) et un segment de connexion (6, 36),
dans lequel le segment de contact (7, 40) est en contact surfacique avec un objet de mesure (20, 21, 2, 3) du capteur électrochimique (10) de telle sorte qu'un contact thermique est établi entre le segment de contact (7, 40) et l'objet de mesure (20, 21, 2, 3),
dans lequel l'objet de mesure (20, 21, 2, 3) est l'électrode de mesure (20), la contre-électrode (21), le moyen de mise en contact électrique (2) pour l'électrode de mesure (20) ou le moyen de mise en contact électrique (3) pour la contre-électrode (21), et
dans lequel l'unité à capteurs de température (6, 7, 8, 9, 36, 37, 38, 39, 40, 51) est configurée pour déterminer au moins approximativement la température de l'électrode de mesure (20) et/ou la température de la contre-électrode (21) en fonction d'une grandeur mesurée corrélée avec la température du segment de contact,
**caractérisé en ce que**
l'unité à capteurs de température (6, 7, 8, 9, 36, 37, 38, 39, 40, 51) comprend en outre un capteur de température (9, 38, 39) et un capteur de tension (8),
dans lequel le segment de contact (7, 40) est en outre connecté de manière électriquement conductrice à l'objet de mesure (20, 21, 2, 3),
dans lequel le segment de connexion (6, 36) connecte électriquement et/ou thermiquement le segment de contact (7, 40) au capteur de température (9, 38, 39),
dans lequel le segment de connexion (6, 36) présente un coefficient Seebeck différent de celui de l'objet de mesure (20, 21, 2, 3),
dans lequel le capteur de tension (8) est configuré pour mesurer une mesure pour une tension thermique [U(Th)] qui survient entre

- le segment de connexion (6, 36) d'une part et
- l'objet de mesure (20, 21) ou le moyen de mise en contact électrique (2, 3) pour l'objet de mesure (20, 21) d'autre part,

dans lequel le capteur de température (9, 38, 39) est configuré pour mesurer, à une position de mesure (P2, P3), en tant que grandeur corrélée avec la température du segment de contact (7, 40), une grandeur corrélée avec la température du segment de connexion (6, 36),
dans lequel la position de mesure (P2, P3) est espacée spatialement des deux électrodes (20, 21) et des deux moyens de mise en contact électriques (2, 3), et
dans lequel l'unité à capteurs de température (6, 7, 8, 9, 36, 37, 38, 39, 40) est configurée pour en fonction

- de la tension thermique [U(Th)] mesurée et
- de la grandeur mesurée qui est corrélée avec la température du segment de connexion (6, 36),

déterminer la température (Temp$_{Ist}$) de l'objet de mesure (20, 21).

**EP 4 303 577 B1**

**2.** Agencement de capteurs (100) selon la revendication 1,
**caractérisé en ce que**

l'objet de mesure est l'électrode de mesure (20) ou la contre-électrode (21),
le segment de connexion (6, 36) présente un coefficient Seebeck différent de celui du moyen de mise en contact électrique (2, 3) de l'objet de mesure (20, 21) et
le capteur de tension (8) est configuré pour mesurer une mesure pour une tension thermique [U(Th)] qui survient entre

- le segment de connexion (6, 36) d'une part et
- le moyen de mise en contact électrique (2, 3) de l'objet de mesure (20, 21) d'autre part.

**3.** Agencement de capteurs (100) selon la revendication 2,
**caractérisé en ce que**
le capteur de température (9, 38, 39) est configuré pour mesurer

- la température du moyen de mise en contact électrique (2, 3) de l'objet de mesure (20, 21), ou
- une grandeur qui est corrélée avec la température du moyen de mise en contact électrique (2, 3) de l'objet de mesure (20, 21),

**4.** Agencement de capteurs (100) selon l'une des revendications précédentes,
**caractérisé en ce que**

l'agencement de capteurs (100) comprend en outre

- un moyen de chauffage (30, 36, 37, 38, 39, 52) pouvant être commandé et
- un appareil de commande (53) traitant les signaux,

dans lequel le moyen de chauffage (30, 36, 37, 38, 39, 52) est configuré pour chauffer l'électrode de mesure (20) et/ou la contre-électrode (21), et
dans lequel l'appareil de commande (53) est configuré

- pour réguler la température réelle ($\text{Temp}_{\text{Ist}}$) de l'électrode de mesure (20) et/ou de la contre-électrode (21) avec pour objectif de régulation

que la température régulée ($\text{Temp}_{\text{Ist}}$) reste dans une plage de températures ($\text{Temp}_{\text{Soll}}$) prédéfinie, et

- pour commander le moyen de chauffage (30, 36, 37, 38, 39, 52) en fonction d'un signal de l'unité à capteurs de température (6, 7, 8, 9, 36, 37, 38, 39, 40, 51) pour la régulation de la température réelle ($\text{Temp}_{\text{Ist}}$).

**5.** Agencement de capteurs (100) selon la revendication 4,
**caractérisé en ce que**

le moyen de chauffage (30, 36, 37, 38, 39, 52) comprend une source de rayonnement (30) pouvant être commandée,
dans lequel la source de rayonnement (30) est configurée pour émettre un rayonnement électromagnétique en direction de l'électrode de mesure (20) et/ou de la contre-électrode (21), et
dans lequel l'appareil de commande (53) est configuré pour, en fonction d'un signal de l'unité à capteurs de température (6, 7, 8, 9, 36, 37, 38, 39, 40, 51), amener l'intensité et/ou l'énergie du rayonnement émis par la source de rayonnement (30) à être réglées à une valeur.

**6.** Agencement de capteurs (100) selon l'une des revendications 4 à 5,
**caractérisé en ce que**

l'appareil de commande est configuré pour calculer, en fonction d'un signal de l'unité à capteurs de température (6, 7, 8, 9, 36, 37, 38, 39, 40, 51), une valeur de consigne pour la tension électrique qui doit être appliquée au moyen de chauffage (30, 36, 37, 38, 39, 52) et
l'agencement de capteurs (100) (100) est configuré pour régler la tension électrique réellement présente au

26

niveau du moyen de chauffage (30, 36, 37, 38, 39, 52) à la valeur de consigne.

7. Agencement de capteurs (100) selon l'une des revendications 4 à 6,
**caractérisé en ce que**

l'agencement de capteurs (100) comprend en outre un moyen de refroidissement pouvant être commandé,
dans lequel le moyen de refroidissement est configuré pour refroidir l'électrode de mesure (20) et/ou la contre-électrode (21), et
dans lequel l'appareil de commande (53) est configuré
pour commander en outre le moyen de refroidissement en fonction d'un signal de l'unité à capteurs de température (6, 7, 8, 9, 36, 37, 38, 39, 40, 51) pour la régulation de la température ($Temp_{Ist}$).

8. Appareil d'analyse comprenant

- un agencement de capteurs (100) selon l'une des revendications précédentes et
- une unité d'entrée,

dans lequel l'unité d'entrée est configurée pour recevoir un échantillon de respiration (Ap) d'un sujet, et
dans lequel l'appareil d'analyse est configuré pour diriger au moins une partie de l'échantillon de respiration (Ap) reçu par l'unité d'entrée vers le capteur électrochimique (10).

9. Procédé permettant l'analyse d'un gaz (Pr) en ce qui concerne au moins un composant gazeux prédéterminé

à l'aide d'un agencement de capteurs (100) qui comprend

- un capteur électrochimique (10) et
- une unité à capteurs de température (6, 7, 8, 9, 36, 37, 38, 39, 40, 51),

dans lequel le capteur électrochimique (10) comprend

- une électrode de mesure (20),
- une contre-électrode (21),
- un moyen de mise en contact électrique (2) pour l'électrode de mesure (20),
- un moyen de mise en contact électrique (3) pour la contre-électrode (21) et
- un électrolyte (28) entre l'électrode de mesure (20) et la contre-électrode (21),

dans lequel l'unité à capteurs de température (6, 7, 8, 9, 36, 37, 38, 39, 40, 51) comprend un élément de mesure (6, 7, 36, 37, 40) électriquement conducteur,
dans lequel l'élément de mesure (6, 7, 36, 37, 40) comprend un segment de contact (7, 40) et un segment de connexion (6, 36),
dans lequel le segment de contact (7, 40) est en contact surfacique avec un objet de mesure (20, 21, 2, 3) du capteur électrochimique (10) de telle sorte qu'un contact thermique est établi entre le segment de contact (7, 40) et l'objet de mesure (20, 21, 2, 3),
dans lequel l'objet de mesure (20, 21, 2, 3) est l'électrode de mesure (20), la contre-électrode (21), le moyen de mise en contact électrique (2) pour l'électrode de mesure (20) ou le moyen de mise en contact électrique (3) pour la contre-électrode (21),
dans lequel le segment de connexion (6, 36) connecte électriquement et/ou thermiquement le segment de contact (7, 40) au capteur de température (9, 38, 39),
dans lequel le procédé comprend les étapes exécutées automatiquement selon lesquelles

- une grandeur de détection est mesurée,

dans lequel la grandeur de détection mesurée est corrélée avec la présence et/ou la concentration du composant gazeux ou d'au moins un composant gazeux,

- une grandeur qui est corrélée avec la température du segment de contact (7, 40) est mesurée et
- l'unité à capteurs de température (6, 7, 8, 9, 36, 37, 38, 39, 40, 51) détermine au moins approximativement la température de l'électrode de mesure (20) et/ou la température de la contre-électrode (21) en fonction de la grandeur mesurée corrélée avec la température du segment de contact,

**caractérisé en ce que**
l'unité à capteurs de température (6, 7, 8, 9, 36, 37, 38, 39, 40, 51) comprend en outre un capteur de température (9, 38, 39) et un capteur de tension (8),
dans lequel le segment de contact (7, 40) est en outre connecté de manière électriquement conductrice à l'objet de mesure (20, 21, 2, 3),
dans lequel le segment de connexion (6, 36) connecte électriquement et/ou thermiquement le segment de contact (7, 40) au capteur de température (9, 38, 39),
dans lequel le segment de connexion (6, 36) présente un coefficient Seebeck différent de celui de l'objet de mesure (20, 21, 2, 3),
dans lequel le procédé comprend en outre les étapes exécutées automatiquement selon lesquelles
le capteur de température (9, 38, 39) mesure, à une position de mesure (P2, P3), en tant que grandeur corrélée avec la température du segment de contact (7, 40), une grandeur corrélée avec la température du segment de connexion (6, 36),
dans lequel la position de mesure (P2, P3) est spatialement espacée des deux électrodes (20, 21) et des deux moyens de mise en contact électriques (2, 3),
le capteur de tension (8) mesure une mesure pour une tension thermique [U(Th)] qui survient entre

- le segment de connexion (6, 36) d'une part et
- l'objet de mesure (20, 21) ou le moyen de mise en contact électrique (2, 3) pour l'objet de mesure (20, 21) d'autre part

et
l'unité à capteurs de température (6, 7, 8, 9, 36, 37, 38, 39, 40, 51) détermine la température ($Temp_{Ist}$) de l'objet de mesure (20, 21) en fonction

- de la tension thermique [U(Th)] mesurée et
- de la grandeur mesurée qui est corrélée avec la température du segment de connexion (6, 36),.

10. Procédé selon la revendication 9,
    **caractérisé en ce que**

    l'agencement de capteurs (100) comprend en outre

    - un moyen de chauffage (30, 36, 37, 38, 39, 52) pouvant être commandé et
    - un appareil de commande (53) traitant les signaux

    et
    le procédé comprend en outre l'étape exécutée automatiquement selon laquelle
    l'appareil de commande (53) régule la température réelle ($Temp_{Ist}$) de l'électrode de mesure (20) et/ou de la contre-électrode (21) avec pour objectif de régulation que ladite température réelle ($Temp_{Ist}$) reste dans une plage de températures ($Temp_{Soll}$) prédéfinie,
    dans lequel, lorsque la température réelle ($Temp_{Ist}$) déterminée est inférieure à la plage de températures ($Temp_{Soll}$),

    - l'appareil de commande (53) active le moyen de chauffage (30, 36, 37, 38, 39, 52),
    - le moyen de chauffage (30, 36, 37, 38, 39, 52) activé chauffe l'électrode de mesure (20) et/ou la contre-électrode (21) et
    - l'appareil de commande (53) désactive à nouveau ultérieurement le moyen de chauffage (30, 36, 37, 38, 39, 52).

FIG. 1

FIG. 2

EP 4 303 577 B1

FIG. 3

31

FIG. 4

FIG. 5

FIG. 6

**FIG. 7**

EP 4 303 577 B1

FIG. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20150076007 A1
- US 3518179 A

- DE 102017008008 A1
- DE 102019003021 A1